# EUROPEAN PATENT SPECIFICATION

(11) **EP 1 572 683 B1**
(45) Date of publication and mention of the grant of the patent: **18.10.2006**
(21) Application number: 03781232.8
(22) Date of filing: 18.12.2003
(51) Int. Cl.: C07D 405/06, C07D 405/12, A61K 31/4468, A61K 31/4523, A61P 1/00, A61P 11/00, A61P 17/00, A61P 19/00

(54) **NOVEL PIPERIDINE DERIVATIVES AS MODULATORS OF CHEMOKINE RECEPTOR CCR5**
NEUE PIPERIDINDERIVATE ALS MODULATOREN VONCHEMOKINREZEPTOR CCR5
NOUVEAUX DERIVES DE PIPERIDINE EN TANT QUE MODULATEURS DU RECEPTEUR CCR5 DE LA CHIMIOKINE

(30) Priority: 20.12.2002 SE 0203820
(43) Date of publication of application: 14.09.2005
(73) Proprietor: AstraZeneca AB, 151 85 Södertälje (SE)
(72) Inventor: OLDFIELD, John, Macclesfield, Cheshire SK10 4TG (GB); TUCKER, Howard, Macclesfield, Cheshire SK10 4TG (GB)
(86) International application number: PCT/SE2003/002005
(87) International publication number: WO 2004/056808

(56) References cited:
- EP-A1- 1 013 276
- EP-A2- 0 903 349
- WO-A1-01/87839
- WO-A1-01/92227
- WO-A1-02/079156

## Description

The present invention relates to heterocyclic derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO01/87839, EP-A1-1013276, WO00/08013, WO99/38514, WO99/04794 and WO00/53600.

Chemokines are chemotactic cytokines that are released by a wide variety of cells to attract macrophages, T cells, eosinophils, basophils and neutrophils to sites of inflammation and also play a rôle in the maturation of cells of the immune system. Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or α) and Cys-Cys (C-C, or β) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

The CCR5 receptor is expressed on T-lymphocytes, monocytes, macrophages, dendritic cells, microglia and other cell types. These detect and respond to several chemokines, principally "regulated on activation normal T-cell expressed and secreted" (RANTES), macrophage inflammatory proteins (MIP) MIP-1α and MIP-1β and monocyte chemoattractant protein-2 (MCP-2).

This results in the recruitment of cells of the immune system to sites of disease. In many diseases it is the cells expressing CCR5 which contribute, directly or indirectly, to tissue damage. Consequently, inhibiting the recruitment of these cells is beneficial in a wide range of diseases.

CCR5 is also a co-receptor for HIV-1 and other viruses, allowing these viruses to enter cells. Blocking the receptor with a CCR5 antagonist or inducing receptor internalisation with a CCR5 agonist protects cells from viral infection.

The present invention provides a compound of formula (I): 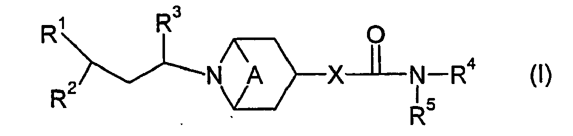 wherein
A is absent or is (CH₂)₂;
R¹ is heterocyclyl wherein the heterocyclyl group is selected from pyran, piperidine, piperazine, pyrrolidine or azetidine and R¹ is optionally mono-substituted by C₁₋₆ alkyl, C₃₋₇ cycloalkyl, phenyl {optionally substituted by halo, C₁₋₄ alkyl, C₁₋₄ alkoxy, CF₃ or OCF₃}, S(O)₂(C₁₋₄ alkyl), S(O)₂(C₁₋₄ fluoroalkyl), S(O)₂phenyl {optionally substituted by halo, cyano, C₁₋₄alkyl, C₁₋₄ alkoxy, CF₃, OCF₃, S(O)₂(C₁₋₄ alkyl) or S(O)₂(C₁₋₄ fluoroalkyl)}, benzyl {optionally substituted by halo, C₁₋₄alkyl, C₁₋₄ alkoxy, CF₃ or OCF₃}, C(O)H, C(O) (C₁₋₄ alkyl), benzoyl {optionally substituted by halo, C₁₋₄ alkyl, C₁₋₄ alkoxy, CF₃ or OCF₃}, C(O)₂(C₁₋₄ alkyl), C(O)NH₂, C(O)NH(C₁₋₄ alkyl) or C(O)NHphenyl {optionally substituted by halo, C₁₋₄ alkyl, C₁₋₄ alkoxy, CF₃ or OCF₃}, or R¹ is phenyl optionally substituted by S(O)₂C₁₋₄ alkyl;
R² is phenyl optionally substituted by halo, C₁₋₄ alkyl, C₁₋₄ alkoxy, S(O)ₙ(C₁₋₄ alkyl), nitro, cyano or CF₃; wherein n is 0, 1 or 2;
R³ is H;
R⁴ is phenyl or benzyl, either of which is optionally substituted by halo, C₁₋₄ alkyl, C₁₋₄ alkoxy, S(O)_{z}(C₁₋₄ alkyl), nitro, cyano or CF₃; wherein z is 0, 1 or 2;
R⁵ is H;
X is CH₂, (CH₂)₂, CH=CH, OCH₂ or S(O)ₙCH₂;
n is 0, 1 or 2;
or a pharmaceutically acceptable salt thereof or a solvate thereof.

Certain compounds of the present invention can exist in different isomeric forms, namely as enantiomers, diastereomers, geometric isomers or tautomers. The present invention covers all such isomers and mixtures thereof in all proportions.

Suitable salts include acid addition salts such as a hydrochloride, hydrobromide, phosphate, acetate, fumarate, maleate, tartrate, citrate, oxalate, methanesulphonate or *p-*toluenesulphonate.

The compounds of the invention may exist as solvates (such as hydrates) and the present invention covers all such solvates.

Alkyl groups and moieties are straight or branched chain and, for example, comprise one to six (such as one to four) carbon atoms. Alkyl is, for example, methyl, ethyl, n-propyl, iso-propyl, n-butyl, sec-butyl or tert-butyl. Methyl is sometimes abbreviated to Me hereinbelow.

Fluoroalkyl includes, for example, one to six, such as one to three, fluorine atoms, and comprises, for example, a CF₃ group. Fluoroalkyl is, for example, CF₃ or CH₂CF₃.

Cycloalkyl is, for example, cyclopropyl, cyclopentyl or cyclohexyl.

In one aspect of the invention R¹ is optionally substituted heterocyclyl, such as optionally substituted: piperidin-1-yl, piperidin-4-yl, piperazin-1-yl, pyrrolidin-1-yl, pyrrolidin-3-yl, azetidin-1-yl or azetidin-3-yl. Said heterocyclyl rings are optionally substituted as described above.

In a further aspect of the invention the heterocyclyl of R¹ is mono-substituted by C₁₋₆ alkyl, C₃₋₇ cycloalkyl, phenyl {optionally substituted by halo (for example fluoro), C₁₋₄ alkyl (for example methyl), C₁₋₄ alkoxy (for example methoxy), CF₃ or OCF₃}, S(O)₂(C₁₋₄ alkyl) (for example S(O)₂CH₃, S(O)₂CH₂CH₃ or S(O)₂CH(CH₃)₂), S(O)₂(C₁₋₄ fluoroalkyl) (for example S(O)₂CF₃ or S(O)₂CH₂CF₃), S(O)₂phenyl {optionally substituted (such as mono-substituted) by halo (for example chloro), cyano, C₁₋₄ alkyl, C₁₋₄ alkoxy, CF₃, OCF₃, S(O)₂(C₁₋₄ alkyl) (for example S(O)₂CH₃ or S(O)₂CH₂CH₂CH₃) or S(O)₂(C₁₋₄ fluoroalkyl) (for example S(O)₂CH₂CF₃)}, benzyl {optionally substituted by halo (for example chloro or fluoro), C₁₋₄ alkyl, C₁₋₄ alkoxy (for example methoxy), CF₃ or OCF₃}, C(O)H, C(O)(C₁₋₄ alkyl), benzoyl {optionally substituted by halo (for example chloro or fluoro), C₁₋₄ alkyl (for example methyl), C₁₋₄ alkoxy, CF₃ or OCF₃}, C(O)₂(C₁₋₄ alkyl), C(O)NH₂, C(O)NH(C₁₋₄ alkyl) or C(O)NHphenyl {optionally substituted by halo (for example fluoro), C₁₋₄ alkyl, C₁₋₄ alkoxy, CF₃ or OCF₃}. In a still further aspect when said heterocyclyl is a 4-substituted piperidin-1-yl, a 1-substituted piperidin-4-yl, a 1-substituted piperazin-1-yl, a 3-substituted pyrrolidin-1-yl, a 1-substituted pyrrolidin-3-yl, a 3-substituted azetidin-1-yl or a 1-substituted azetidin-3-yl.

In another aspect of the invention R¹ is piperidinyl (such as piperidin-4-yl) or piperazinyl (such as piperazin-1-yl) substituted (such as N-substituted) by S(O)₂C₁₋₄ alkyl (such as S(O)₂CH₃), S(O)₂C₁₋₄ fluoroalkyl (such as S(O)₂CF₃) or C(O)NH-phenyl (wherein the phenyl is optionally substituted by halo, for example fluoro). In a further aspect of the invention R¹ is phenyl substituted by S(O)₂C₁₋₄ alkyl (such as S(O)₂CH₃).

In a still further aspect R² is optionally substituted (for example unsubstituted or substituted in the 2-, 3-, or 3- and 5- positions) phenyl (such as optionally substituted by halo (such as chloro or fluoro), cyano, methyl, ethyl, methoxy, ethoxy or CF₃).

In another aspect of the invention R² is optionally substituted (for example unsubstituted or substituted in the 2-, 3-, or 3- and 5- positions) phenyl (such as optionally substituted by halo (for example chloro or fluoro)). In a further aspect of the invention R² is phenyl optionally substituted by 0, 1 or 2 fluorines. For example R² is phenyl, 3-fluorophenyl, 3-chlorophenyl, 3,5-difluorophenyl. In a further aspect of the invention R² is phenyl.

In yet another aspect of the invention R⁴ is phenyl or benzyl either of which is optionally substituted by halo (for example fluoro) or S(O)₂(C₁₋₄ alkyl) (for example S(O)₂CH₃).

In yet another aspect of the invention A is absent.

In a further aspect X is CH₂ or CH=CH.

In yet another aspect the present invention provides a compound of formula (Ia): 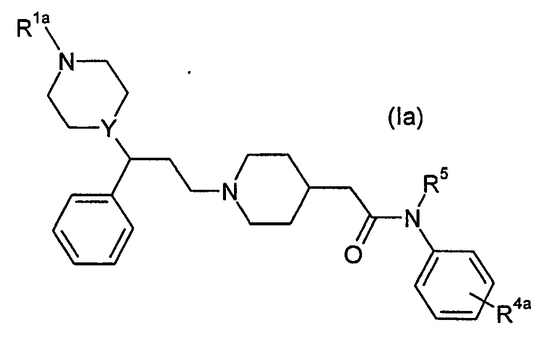 wherein Y is CH or N; R^{4a} is as defined for an optional substituent on optionally substituted phenyl (above); R^{1a} is mono-substituted by C₁₋₆ alkyl, C₃₋₇ cycloalkyl, phenyl {optionally substituted by halo (for example fluoro), C₁₋₄ alkyl (for example methyl), C₁₋₄ alkoxy (for example methoxy), CF₃ or OCF₃}, S(O)₂(C₁₋₄ alkyl) (for example S(O)₂CH₃, S(O)₂CH₂CH₃ or S(O)₂CH(CH₃)₂), S(O)₂(C₁₋₄ fluoroalkyl) (for example S(O)₂CF₃ or S(O)₂CH₂CF₃), S(O)₂phenyl {optionally substituted (such as mono-substituted) by halo (for example chloro), cyano, C₁₋₄ alkyl, C₁₋₄ alkoxy, CF₃, OCF₃, S(O)₂(C₁₋₄ alkyl) (for example S(O)₂CH₃ or S(O)₂CH₂CH₂CH₃) or S(O)₂(C₁₋₄ fluoroalkyl) (for example S(O)₂CH₂CF₃)}, benzyl {optionally substituted by halo (for example chloro or fluoro), C₁₋₄ alkyl, C₁₋₄ alkoxy (for example methoxy), CF₃ or OCF₃}, C(O)H, C(O)(C₁₋₄ alkyl), benzoyl {optionally substituted by halo (for example chloro or fluoro), C₁₋₄ alkyl (for example methyl), C₁₋₄, alkoxy, CF₃ or OCF₃}, C(O)₂(C₁₋₄ alkyl), C(O)NH₂, C(O)NH(C₁₋₄ alkyl) or C(O)NHphenyl {optionally substituted by halo (for example fluoro), C₁₋₄ alkyl, C₁₋₄ alkoxy, CF₃ or OCF₃}; and R⁵ is hydrogen.

In a further aspect the present invention provides a compound of formula (Ib): 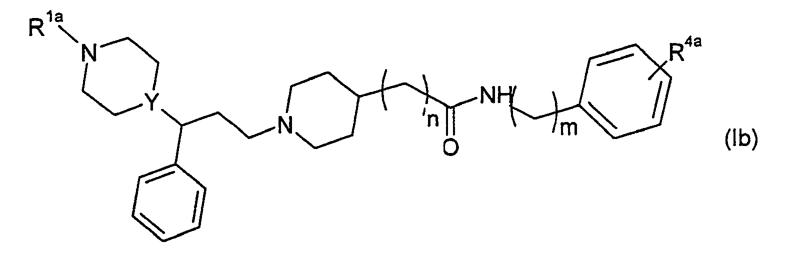 wherein R^{1a}, R^{4a} and Y are as defined above; n is 1 or 2; and m is 0 or 1.

In a still further aspect the present invention provides a compound of formula (Ic): 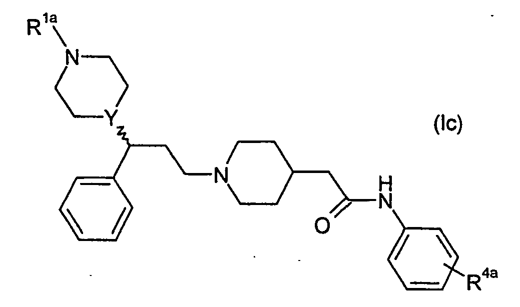 wherein R^{1a}, R^{4a} and Y are as defined above.

In another aspect the present invention provides a compound of formula (Id): 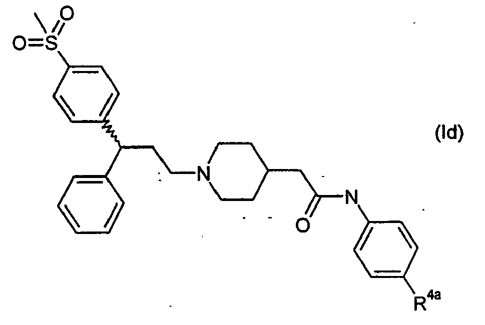 wherein R^{4a} is as defined above.

In yet another aspect the present invention provides a compound of formula (Ie): 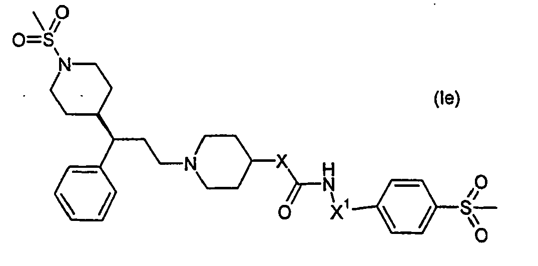 wherein X is as defined above; and X¹ is CH₂ or is absent.

The compounds listed in Tables I to V illustrate the invention.

### Table I

Table I comprises compounds of formula (Ia):

| | | | | |
|---|---|---|---|---|
| 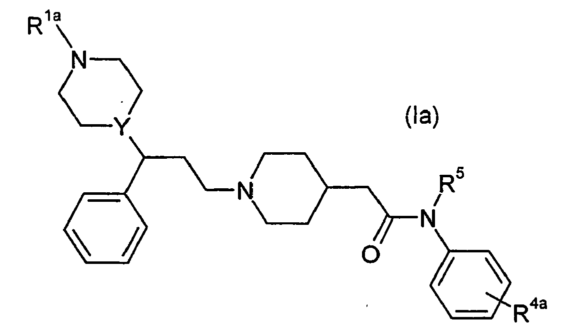 | | | | |

| Compound No | Y | R^{1a} | R⁵ | R^{4a} |
|---|---|---|---|---|
| 1 | CH | methanesulphonyl | H | 4-methanesulphonyl |
| 2 | CH | trifluoromethanesulphonyl | H | 4-methanesulphonyl |
| 3 | N | phenyl | H | 4-methanesulphonyl |
| 4 | CH | (2,5-difluorophenyl)aminocarbonyl | H | 4-methanesulphonyl |
| 5 | CH | 4-chlorobenzoyl | H | 4-methanesulphonyl |
| 6 | CH | methanesulphonyl | H | 4-fluoro |
| 7 | CH | methanesulphonyl | H | 3,5-difluoro |
| 9 | CH | methanesulphonyl | H | 4-chloro |
| 12 | CH | methanesulphonyl | H | 4-methyl |
| 13 | CH | methanesulphonyl | H | 4-methoxy |
| 14 | CH | methanesulphonyl | H | 4-trifluormethyl |

### Table II

Table II comprises compounds of formula (Ib):

| | | | | | |
|---|---|---|---|---|---|
| 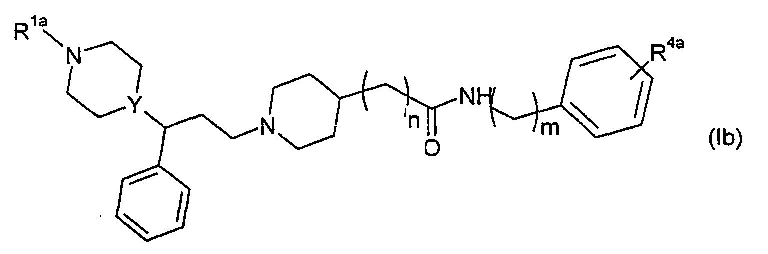 | | | | | |

| Compound No | R^{1a} | Y | n | m | R^{4a} |
|---|---|---|---|---|---|
| 1 | methanesulphonyl | CH | 1 | 1 | 4-methanesulphonyl |
| 2 | trifluoromethanesulphonyl | CH | 1 | 1 | 4-methanesulphonyl |
| 3 | trifluoromethanesulphonyl | CH | 1 | 1 | 4-methanesulphonyl |

### Table III

Table III comprises compounds of formula (Ic):

| | | | | |
|---|---|---|---|---|
| 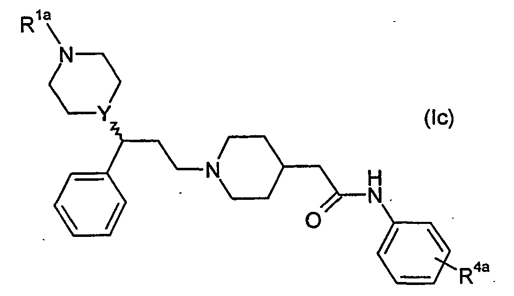 | | | | |

| Compound No | R^{1a} | Y | stereochemistry | R^{4a} |
|---|---|---|---|---|
| 1 | methanesulphonyl | CH | R or S | 4-methanesulphonyl |
| 2 | methanesulphonyl | CH | S or R | 4-methanesulphonyl |
| 3 | trifluoromethanesulphonyl | CH | S or R | 4-methanesulphonyl |
| 4 | trifluoromethanesulphonyl | CH | R or S | 4-methanesulphonyl |
| 5 | methanesulphonyl | CH | R or S | 4-chloro |
| 6 | methanesulphonyl | CH | R or S | 3,5-difluoro |
| 7 | methanesulphonyl | CH | S or R | 4-fluoro |
| 8 | methanesulphonyl | CH | R or S | 4-fluoro |
| 9 | trifluoromethanesulphonyl | N | S or R | 4-fluoro |
| 10 | methanesulphonyl | N | S or R | 4-methanesulphonyl |
| 11 | trifluoromethanesulphonyl | N | S or R | 4-methanesulphonyl |

### Table IV

Table IV comprises compounds of formula (Id):

| | | |
|---|---|---|
| 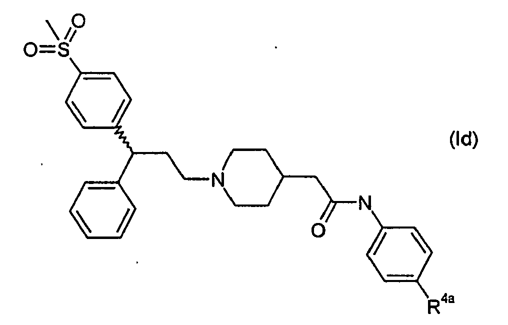 | | |

| Compound No | stereochemistry | R^{4a} |
|---|---|---|
| 1 | S | 4-methanesulphonyl |
| 2 | S | 4-chloro |
| 3 | S | 4-fluoro |

### Table V

Table V comprises compounds of formula (Ie):

| | | |
|---|---|---|
| 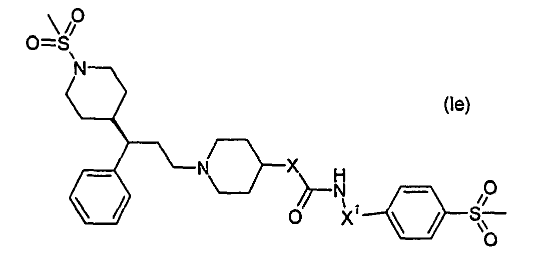 | | |

| Compound No | X | X¹ |
|---|---|---|
| 1 | CH=CH | CH₂ |
| 2 | CH₂CH₂ | - |
| 3 | CH₂CH₂ | CH₂ |

In yet another aspect the invention provides each individual compound listed in the tables above.

The compounds of formula (I), (Ia), (Ib), (Ic), (Id) and (Ie) can be prepared as shown below.

A compound of the invention wherein R¹ is an N-linked optionally substituted heterocycle can be prepared by reacting a compound of formula (II): 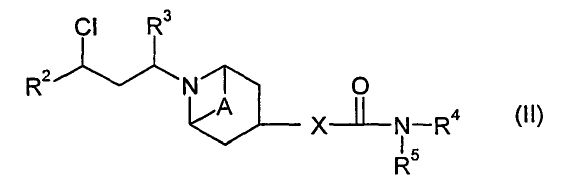 wherein R², R³, R⁴, R⁵, A and X are as defined above, with a compound R¹H (wherein the H is on a heterocycle ring nitrogen atom) wherein R¹ is as defined above, in the presence of a suitable base (for example a tri(C₁₋₆ alkyl)amine such as triethylamine or Hunig's base), in a suitable solvent (such as a chlorinated solvent, for example dichloromethane) and, for example, at a room temperature (for example 10-30°C), optionally in the presence of sodium iodide.

A compound of the invention, wherein R³ is hydrogen, can be prepared by coupling a compound of formula (III): 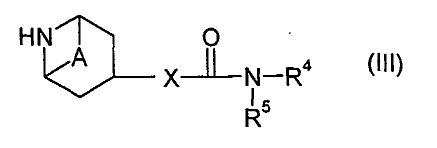 wherein R⁴, R⁵, A and X are as defined above, with a compound of formula (IV): 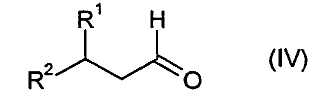 wherein R¹ and R² are as defined above, in the presence of NaBH(OAc)₃ (wherein Ac is C(O)CH₃) in a suitable solvent (such as a chlorinated solvent, for example dichloromethane) at room temperature (for example 10-30°C).

Alternatively, compounds of the invention can be prepared according to Schemes 1-3,5-7 (below).

Alternatively, compounds of the invention can be prepared by using or adapting methods described in WO01/87839, EP-A1-1013276, WO00/08013, WO99/38514, WO99/04794, WO00/76511, WO00/76512, WO00/76513, WO00/76514, WO00/76972 or US 2002/0094989.

The starting materials for these processes are either commercially available or can be prepared by literature methods, adapting literature methods or by following or adapting Methods herein described.

In a still further aspect the invention provides processes for preparing the compounds of formula (I), (Ia), (Ib), (Ic), (Id) and (Ie). Many of the intermediates in the processes are novel and these are provided as further features of the invention.

The compounds of the invention have activity as pharmaceuticals, in particular as modulators (such as agonists, partial agonists, inverse agonists or antagonists) of chemokine receptor (especially CCR5) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

The compounds of the present invention are also of value in inhibiting the entry of viruses (such as human immunodeficiency virus (HIV)) into target calls and, therefore, are of value in the prevention of infection by viruses (such as HIV), the treatment of infection by viruses (such as HIV) and the prevention and/or treatment of acquired immune deficiency syndrome (AIDS).

According to a further feature of the invention there is provided a compound of the formula (I), (Ia), (Ib), (Ic), (Id) or (Ie), or a pharmaceutically acceptable salt thereof or a solvate thereof, for use in a method of treatment of a warm blooded animal (such as man) by therapy (including prophylaxis).

According to a further feature of the present invention there is provided a method for modulating chemokine receptor activity (especially CCR5 receptor activity) in a warm blooded animal, such as man, in need of such treatment, which comprises administering to said animal an effective amount of a compound of the present invention, or a pharmaceutically acceptable salt thereof or a solvate thereof.

The present invention also provides the use of a compound of the formula (I), (Ia), (Ib), (Ic), (Id) or (Ie), or a pharmaceutically acceptable salt thereof or a solvate thereof, as a medicament, especially a medicament for the treatment of transplant rejection, respiratory disease, psoriasis or rheumatoid arthritis (especially rheumatoid arthritis). [Respiratory disease is, for example, COPD, asthma {such as bronchial, allergic, intrinsic, extrinsic or dust asthma, particularly chronic or inveterate asthma (for example late asthma or airways hyper-responsiveness)} or rhinitis {acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis}; and is particularly asthma or rhinitis].

In another aspect the present invention provides the use of a compound of the formula (I), (Ia), (Ib), (Ic), (Id) or (Ie), or a pharmaceutically acceptable salt thereof or a solvate thereof, in the manufacture of a medicament for use in therapy (for example modulating chemokine receptor activity (especially CCR5 receptor activity (especially rheumatoid arthritis)) in a warm blooded animal, such as man).

The invention also provides a compound of the formula (I), (Ia), (Ib), (Ic), (Id) or (Ie), or a pharmaceutically acceptable salt thereof or a solvate thereof, for use as a medicament, especially a medicament for the treatment of rheumatoid arthritis.

In another aspect the present invention provides the use of a compound of the formula (I), (Ia), (Ib), (Ic), (Id) or (Ie), or a pharmaceutically acceptable salt thereof or a solvate thereof, in the manufacture of a medicament for use in therapy (for example modulating chemokine receptor activity (especially CCR5 receptor activity (especially rheumatoid arthritis)) in a warm blooded animal, such as man).

The invention further provides the use of a compound of formula (I), (Ia), (Ib), (Ic), (Id) or (Ie), or a pharmaceutically acceptable salt thereof, in the manufacture of a medicament for use in the treatment of:
(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis; sarcoidosis; farmer's lung and related diseases; nasal polyposis; fibroid lung or idiopathic interstitial pneumonia;
(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;
(3) (skin and eyes) psoriasis, atopic dermatitis, contact dermatitis or other eczmatous dermitides, seborrhoetic dermatitis, Lichen planus, Phemphigus, bullous Phemphigus, Epidermolysis bullosa, urticaria, angiodermas, vasculitides erythemas, cutaneous eosinophilias, uveitis, Alopecia areata or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), Lupus disorders (such as lupus erythematosus or systemic lupus), erythematosus; Hashimoto's thyroiditis, myasthenia gravis, type I diabetes, nephrotic syndrome, eosinophilia fascitis, hyper IgE syndrome, leprosy (such as lepromatous leprosy), Peridontal disease, Sezary syndrome, idiopathic thrombocytopenia pupura or disorders of the menstrual cycle;
in a warm blooded animal, such as man.

The present invention further provides a method of treating a chemokine mediated disease state (especially a CCR5 mediated disease state) in a warm blooded animal, such as man, which comprises administering to a mammal in need of such treatment an effective amount of a compound of formula (I), (Ia), (Ib), (Ic), (Id) or (Ie), or a pharmaceutically acceptable salt thereof or solvate thereof.

In order to use a compound of the invention, or a pharmaceutically acceptable salt thereof or solvate thereof, for the therapeutic treatment of a warm blooded animal, such as man, in particular modulating chemokine receptor (for example CCR5 receptor) activity, said ingredient is normally formulated in accordance with standard pharmaceutical practice as a pharmaceutical composition.

Therefore in another aspect the present invention provides a pharmaceutical composition which comprises a compound of the formula (I), (Ia), (Ib), (Ic), (Id) or (Ie), or a pharmaceutically acceptable salt thereof or a solvate thereof (active ingredient), and a pharmaceutically acceptable adjuvant, diluent or carrier. In a further aspect the present invention provides a process for the preparation of said composition which comprises mixing active ingredient with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art into the form of, for example, aerosols, dry powder formulations, tablets, capsules, syrups, powders, granules, aqueous or oily solutions or suspensions, (lipid) emulsions, dispersible powders, suppositories, ointments, creams, drops and sterile injectable aqueous or oily solutions or suspensions.

A suitable pharmaceutical composition of this invention is one suitable for oral administration in unit dosage form, for example a tablet or capsule which contains between 0.1mg and 1g of active ingredient.

In another aspect a pharmaceutical composition of the invention is one suitable for intravenous, subcutaneous or intramuscular injection.

Each patient may receive, for example, an intravenous, subcutaneous or intramuscular dose of 0.01mgkg⁻¹ to 100mgkg⁻¹ of the compound, preferably in the range of 0.1mgkg⁻¹ to 20mgkg⁻¹ of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose, the composition being administered 1 to 4 times per day.

The following illustrate representative pharmaceutical dosage forms containing the compound of formula (I), (Ia), (Ib), (Ic), (Id) or (Ie), or a pharmaceutically acceptable salt thereof or a solvent thereof (hereafter Compound X), for therapeutic or prophylactic use in humans:
(a)

| Tablet I | mg/tablet |
|---|---|
| Compound X | 100 |
| Lactose Ph.Eur. | 179 |
| Croscarmellose sodium | 12.0 |
| Polyvinylpyrrolidone | 6 |
| Magnesium stearate | 3.0 |

(b)

| Tablet II | mg/tablet |
|---|---|
| Compound X | 50 |
| Lactose Ph.Eur. | 229 |
| Croscarmellose sodium | 12.0 |
| Polyvinylpyrrolidone | 6 |
| Magnesium stearate | 3.0 |

(c)

| Tablet III | mg/tablet |
|---|---|
| Compound X | 1.0 |
| Lactose Ph.Eur. | 92 |
| Croscarmellose sodium | 4.0 |
| Polyvinylpyrrolidone | 2.0 |
| Magnesium stearate | 1.0 |

(d)

| Capsule | mg/capsule |
|---|---|
| Compound X | 10 |
| Lactose Ph.Eur. | 389 |
| Croscarmellose sodium | 100 |
| Magnesium stearate | 1.0 |

(e)

| Injection I | (50 mg/ml) |
|---|---|
| Compound X | 5.0% w/v |
| Isotonic aqueous solution | to 100% |

Buffers, pharmaceutically-acceptable cosolvents such as polyethylene glycol, polypropylene glycol, glycerol or ethanol or complexing agents such as hydroxy-propyl β-cyclodextrin may be used to aid formulation.

The above formulations may be obtained by conventional procedures well known in the pharmaceutical art. The tablets (a)-(c) may be enteric coated by conventional means, for example to provide a coating of cellulose acetate phthalate.

The invention further relates to combination therapies or compositions wherein a compound of formula (I), or a pharmaceutically acceptable salt, solvate or a solvate of a salt thereof, or a pharmaceutical composition comprising a compound of formula (I), or a pharmaceutically acceptable salt, solvate or a solvate of a salt thereof, is administered concurrently (possibly in the same composition) or sequentially with an agent for the treatment of any one of the above disease states.

In particular, for the treatment of the inflammatory diseases rheumatoid arthritis, psoriasis, inflammatory bowel disease, COPD, asthma and allergic rhinitis a compound of the invention can be combined with a TNF-α inhibitor (such as an anti-TNF monoclonal antibody (such as Remicade, CDP-870 and D.sub2.E.sub7.), or a TNF receptor immunoglobulin molecule (such as Enbrel.reg.)), a non-selective COX-1 / COX-2 inhibitor (such as piroxicam or diclofenac; a propionic acid such as naproxen, flubiprofen, fenoprofen, ketoprofen or ibuprofen; a fenamate such as mefenamic acid, indomethacin, sulindac or apazone; a pyrazolone such as phenylbutazone; or a salicylate such as aspirin), a COX-2 inhibitor (such as meloxicam, celecoxib, rofecoxib, valdecoxib or etoricoxib) low dose methotrexate, lefunomide; ciclesonide; hydroxychloroquine, d-penicillamine or auranofin, or parenteral or oral gold.

The present invention still further relates to the combination of a compound of the invention together with:
- a leukotriene biosynthesis inhibitor, a 5-lipoxygenase (5-LO) inhibitor or a 5-lipoxygenase activating protein (FLAP) antagonist, such as zileuton, ABT-761, fenleuton, tepoxalin, Abbott-79175, Abbott-85761, an N-(5-substituted)-thiophene-2-alkylsulfonamide, a 2,6-di-tert-butylphenol hydrazones, a methoxytetrahydropyran such as Zeneca ZD-2138, SB-210661, a pyridinyl-substituted 2-cyanonaphthalene compound such as L-739,010; a 2-cyanoquinoline compound such as L-746,530; an indole or quinoline compound such as MK-591, MK-886 or BAY x 1005;
- a receptor antagonist for a leukotriene LTB.sub4., LTC.sub4., LTD.sub4. or LTE.sub4. selected from the group consisting of a phenothiazin-3-one such as L-651,392; an amidino compound such as CGS-25019c; a benzoxalamine such as ontazolast; a benzenecarboximidamide such as BIIL 284/260; or a compound such as zafirlukast, ablukast, montelukast, pranlukast, verlukast (MK-679), RG-12525, Ro-245913, iralukast (CGP 45715A) or BAY x 7195;
- a PDE4 inhibitor including an inhibitor of the isoform PDE4D;
- an antihistaminic H.sub1. receptor antagonist such as cetirizine, loratadine, desloratadine, fexofenadine, astemizole, azelastine or chlorpheniramine;
- a gastroprotective H.sub2. receptor antagonist;
- an α.sub1.- and α.sub2.-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride or ethylnorepinephrine hydrochloride;
- an anticholinergic agent such as ipratropium bromide, tiotropium bromide, oxitropium bromide, pirenzepine or telenzepine;
- a β.sub1.- to β.sub4.-adrenoceptor agonist such as metaproterenol, isoproterenol, isoprenaline, albuterol, salbutamol, formoterol, salmeterol, terbutaline, orciprenaline, bitolterol mesylate or pirbuterol, or a methylxanthanine including theophylline and aminophylline; sodium cromoglycate; or a muscarinic receptor (M1, M2, and M3) antagonist;
- an insulin-like growth factor type I (IGF-1) mimetic;
- an inhaled glucocorticoid with reduced systemic side effects, such as prednisone, prednisolone, flunisolide, triamcinolone acetonide, beclomethasone dipropionate, budesonide, fluticasone propionate or mometasone furoate;
- an inhibitor of a matrix metalloprotease (MMP), such as a stromelysin, a collagenase, or a gelatinase or aggrecanase; such as collagenase-1 (MMP-1), collagenase-2 (MMP-8), collagenase-3 (MMP-13), stromelysin-1 (MMP-3), stromelysin-2 (MMP-10), and stromelysin-3 (MMP-11) or MMP-12;
- a modulator of chemokine receptor function such as CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family;
- an osteoporosis agent such as roloxifene, droloxifene, lasofoxifene or fosomax;
- an immunosuppressant agent such as FK-506, rapamycin, cyclosporine, azathioprine or methotrexate;
- a compound useful in the treatment of AIDS and/or HIV infection for example: an agent which prevents or inhibits the viral protein gp120 from engaging host cell CD4 {such as soluble CD4 (recombinant); an anti-CD4 antibody (or modified / recombinant antibody) for example PRO542; an anti-group 120 antibody (or modified / recombinant antibody); or another agent which interferes with the binding of group120 to CD4 for example BMS806}; an agent which prevents binding to a chemokine receptor, other than CCR5, used by the HIV virus {such as a CXCR4 agonist or antagonist or an anti-CXCR4 antibody}; a compound which interferes in the fusion between the HIV viral envelope and a cell membrane {such as an anti-group 41 antibody; enfuvirtide (T-20) or T-1249}; an inhibitor of DC-SIGN (also known as CD209) {such as an anti-DC-SIGN antibody or an inhibitor of DC-SIGN binding}; a nucleoside/nucleotide analogue reverse transciptase inhibitor {for example zidovudine (AZT), nevirapine, didanosine (ddI), zalcitabine (ddC), stavudine (d4T), lamivudine (3TC), abacavir, adefovir or tenofovir (for example as free base or as disoproxil fumarate)}; a non-nucleoside reverse transciptase inhibitor {for example nevirapine, delavirdine or efavirenz}; a protease inhibitor {for example ritonavir, indinavir, saquinavir (for example as free base or as mesylate salt), nelfinavir (for example as free base or as mesylate salt), amprenavir, lopinavir or atazanavir (for example as free base or as sulphate salt)}; a ribonucleotide reductase inhinbitor {for example hydroxyurea}; or an antiretroviral {for example emtricitabine}; or,
- an existing therapeutic agent for the treatment of osteoarthritis, for example a non-steroidal anti-inflammatory agent (hereinafter NSAID's) such as piroxicam or diclofenac, a propionic acid such as naproxen, flubiprofen, fenoprofen, ketoprofen or ibuprofen, a fenamate such as mefenamic acid, indomethacin, sulindac or apazone, a pyrazolone such as phenylbutazone, a salicylate such as aspirin, a COX-2 inhibitor such as celecoxib, valdecoxib, rofecoxib or etoricoxib, an analgesic or intra-articular therapy such as a corticosteroid or a hyaluronic acid such as hyalgan or synvisc, or a P2X7 receptor antagonist.

The present invention still further relates to the combination of a compound of the invention together with: (i) a tryptase inhibitor; (ii) a platelet activating factor (PAF) antagonist; (iii) an interleukin converting enzyme (ICE) inhibitor; (iv) an IMPDH inhibitor; (v) an adhesion molecule inhibitor including a VLA-4 antagonist; (vi) a cathepsin; (vii) a MAP kinase inhibitor; (viii) a glucose-6 phosphate dehydrogenase inhibitor; (ix) a kinin-B.sub1. - and B.sub2. -receptor antagonist; (x) an anti-gout agent, e.g., colchicine; (xi) a xanthine oxidase inhibitor, e.g., allopurinol; (xii) an uricosuric agent, e.g., probenecid, sulfinpyrazone or benzbromarone; (xiii) a growth hormone secretagogue; (xiv) a transforming growth factor (TGFβ); (xv) a platelet-derived growth factor (PDGF); (xvi) a fibroblast growth factor, e.g., basic fibroblast growth factor (bFGF); (xvii) a granulocyte macrophage colony stimulating factor (GM-CSF); (xviii) a capsaicin cream; (xix) a Tachykinin NK.sub1. and NK.sub3. receptor antagonist selected from the group consisting of NKP-608C; SB-233412 (talnetant); and D-4418; (xx) an elastase inhibitors selected from the group consisting of UT-77 and ZD-0892; (xxi) a TNFα converting enzyme inhibitor (TACE); (xxii) an induced nitric oxide synthase inhibitor (iNOS); or (xxiii) a chemoattractant receptor-homologous molecule expressed on TH2 cells (a CRTH2 antagonist).

The invention will now be illustrated by the following non-limiting Examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30 mm Hg) with a bath temperature of up to 60°C;
(iii) chromatography unless otherwise stated means flash chromatography on silica gel; thin layer chromatography (TLC) was carried out on silica gel plates; where a "Bond Elut" column is referred to, this means a column containing 10g or 20g of silica of 40 micron particle size, the silica being contained in a 60ml disposable syringe and supported by a porous disc, obtained from Varian, Harbor City, California, USA under the name "Mega Bond Elut SI". Where an "Isolute^{™} SCX column" is referred to, this means a column containing benzenesulphonic acid (non-endcapped) obtained from International Sorbent Technology Ltd., 1st House, Duffryn Industial Estate, Ystrad Mynach, Hengoed, Mid Glamorgan, UK. Where "Argonaut^{™} PS-*tris*-amine scavenger resin" is referred to, this means a *tris*-(2-aminoethyl)amine polystyrene resin obtained from Argonaut Technologies Inc., 887 Industrial Road, Suite G, San Carlos, California, USA.
(iv) in general, the course of reactions was followed by TLC and reaction times are given for illustration only;
(v) yields, when given, are for illustration only and are not necessarily those which can be obtained by diligent process development; preparations were repeated if more material was required;
(vi) when given, ¹H NMR data is quoted and is in the form of delta values for major diagnostic protons, given in parts per million (ppm) relative to tetramethylsilane (TMS) as an internal standard, determined at 300 MHz using perdeuterio DMSO (CD₃SOCD₃) as the solvent unless otherwise stated; coupling constants (J) are given in Hz;
(vii) chemical symbols have their usual meanings; SI units and symbols are used;
(viii) solvent ratios are given in percentage by volume;
(ix) mass spectra (MS) were run with an electron energy of 70 electron volts in the chemical ionisation (APCI) mode using a direct exposure probe; where indicated ionisation was effected by electrospray (ES); where values for m/z are given, generally only ions which indicate the parent mass are reported, and unless otherwise stated the mass ion quoted is the positive mass ion - (M+H)⁺;
(x) LCMS characterisation was performed using a pair of Gilson 306 pumps with Gilson 233 XL sampler and Waters ZMD4000 mass spectrometer. The LC comprised water symmetry 4.6x50 column C18 with 5 micron particle size. The eluents were: A, water with 0.05% formic acid and B, acetonitrile with 0.05% formic acid. The eluent gradient went from 95% A to 95% B in 6 minutes. Where indicated ionisation was effected by electrospray (ES); where values for m/z are given, generally only ions which indicate the parent mass are reported, and unless otherwise stated the mass ion quoted is the positive mass ion - (M+H)⁺;
(xi) the ACD naming database was used to name the compounds of Example 6 and Method D; and,
(xii) the following abbreviations are used:

| | |
|---|---|
| DMSO | dimethyl sulfoxide; |
| DMF | *N*-dimethylformamide; |
| THF | tetrahydrofuran; and, |
| HBTU | O-(7-Benzotriazol-1-yl)-*N*,*N*,*N',N*'-tetramethyluronium hexafluorophosphate. |

### EXAMPLE 1

This Example illustrates the preparation of N-(4-methanesulphonylphenyl)-1-[3-phenyl-3-(N-trifluoromethanesulphonylpiperidin-4-yl)propyl]piperidine-4-acetamide (Compound No. 2, Table II).

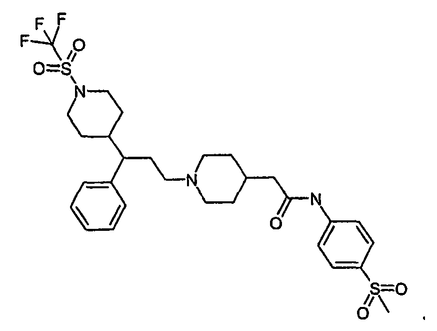

Trifluoroacetic anhydride (51µl) was added to a solution of N-(4-methanesulphonylphenyl)-1-[(3-phenyl-3-piperidin-4-yl)propyl]piperidine-4-acetamide (150mg) and triethylamine (42µl) in dichloromethane (10ml) at-70°C. The reaction mixture was allowed to warm to room temperature and was stirred for 48 hours then washed with 2M sodium hydroxide (15ml) and brine (15ml) and dried. The dichloromethane solution was poured onto a 20g silica Bond Elut column and eluted with a solvent gradient (ethyl acetate-20% methanol/ethyl acetate) to give the title compound, yield 90mg, MH⁺ 630. NMR (CDCl₃): 1.2-1.4 (m; 6H), 1.65 (m, 1H) 1.8(m, 3H) 1.9-2.2 (m, 6H) 2.3 (d, 2H) 2.4 (m, 1H) 2.8 (m, 3H) 3.0 (m, 1H) 3.05 (s, 3H) 3.8 (d, 1H) 4.0 (d, 1H) 7.1 (m, 2H) 7.2 (m, 3H) 7.75 (m, 3H) 7.9 (m, 2H).

### N-(4-Methanesulphonylphenyl)-1-[(3-phenyl-3-piperidin-4-yl)propyl]piperidine-4-acetamide

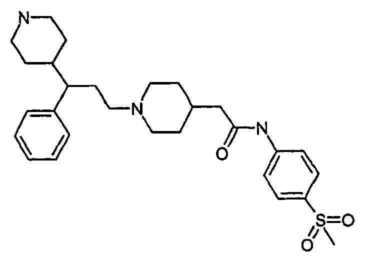

N-(4-Methanesulphonylphenyl)piperidine-4-acetamide (1g) was added to a solution of 3-phenyl-3-(1-tert-butoxycarbonylpiperidin-4-yl)propionaldehyde (0.92g) in dichloromethane (20ml) containing 2 drops of acetic acid. Sodium triacetoxyborohydride (1.074g) was added and the mixture was stirred for 16 hours. The reaction mixture was washed with 2M sodium hydroxide (10ml), water (20ml) and brine (20ml) and dried. Removal of the solvent gave N-(4-methanesulphonylphenyl)-1-[(3-phenyl-3-(1-tert-butoxycarbonylpiperidin-4-yl)propyl]piperidine-4-acetamide, yield 2g, MH⁺ 598.

This material was dissolved in dichloromethane (20ml) and trifluoroacetic anhydride (5ml) was added and the reaction mixture was stirred for 1 hour. The solvent was removed under reduced pressure, the residue was diluted with 2M sodium hydroxide (10ml) and extracted with dichloromethane (2x25ml). The combined dichloromethane extracts were dried and evaporated to dryness to give N-(4-methanesulphonylphenyl)-1-[(3-phenyl-3-piperidin-4-yl)propyl]piperidine-4-acetamide, yield 1.5g, MH⁺ 498.

### EXAMPLE 2

This Example illustrates the preparation of N-(4-methanesulphonylphenyl)-1-[3-phenyl-3-(N-{3,5-difluorophenyl}amino-carbonylpiperidin-4-yl)propyl]piperidine-4-acetamide.(Compound No. 4, Table I).

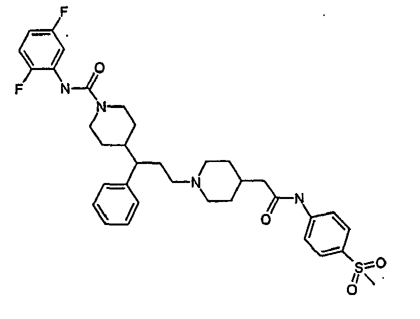

3,5-Difluorophenylisocyanate (47µl) was added to a solution of N-(4-methanesulphonylphenyl)-1-[(3-phenyl-3-piperidin-4-yl)propyl]piperidine-4-acetamide (200mg, Example 1) in dichloromethane (5ml) and the reaction mixture was stirred for 16 hours. The reaction mixture was poured onto a 20g silica Bond Elut column which was eluted with a solvent gradient (ethyl acetate-40% methanol/ethyl acetate) to give the title compound as a white foam, yield 150mg, MH⁺ 653. NMR (CDCl₃): 1.3 (m, 5H) 1.7-1.9 (m, 10H) 2.0-2.1 (m, 4H) 2.4 (m, 1H) 2.8 (m, 3H) 3.0 (s, 3H) 7.1 (m,2H) 7.2-7.4 (m, 7H) 7.7 (m, 2H) 7.9 (d, 2H) 8.0 (bs, 1H).

### EXAMPLE 3

Separation of the enantiomers of N-(4-methanesulphonylphenyl)-1-[3-phenyl-3-(N-methanesulphonylpiperidin-4-yl)propyl]piperidine-4-acetamide (Compound Nos 3 and 4, Table III).

Racemic N-(4-methanesulphonylphenyl)-1-[3-phenyl-3-(N-methanesulphonylpiperidin-4-yl)propyl]piperidine-4-acetamide (240 mg) was chromatographed on a Merck 50mm 20µm Chiralpak AD column (Lot AD00SC-HL001) eluted with a mixture of acetonitrile/methanol (85/15) to give:
Less polar isomer, 85 mg, Compound No. 3 of Table III.
More polar isomer, 86 mg, Compound No. 4 of Table III.

### EXAMPLE 4

This Example illustrates the preparation of (R) or (S) trans N-(4-methanesulphonylphenylmethyl)-1-[3-phenyl-3-(N-methanesulphonylpiperidin-4-yl)propyl]piperidine-4-prop-2-enamide (Compound No. 1 Table V).

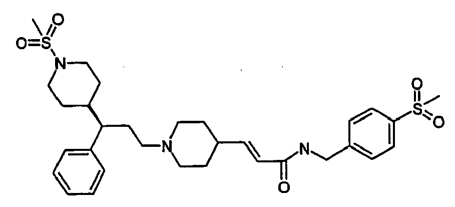

Sodium triacetoxyborohydride (172mg) was added to a solution of 3R 3-[1-hydroxy-3-phenyl-3-(N-methanesulphonylpiperidin-4-yl)propyl]-(4R,5S)-1,5-dimethyl-4-phenyl-2-imidazolidinone (400mg) and 3-(piperidin-4-yl)-N-(4-methanesulphonylphenylmethyl)-acrylamide (242mg) in dichloromethane (25ml) containing 2 drops of acetic acid and the mixture was stirred for 16 hours. The reaction mixture was dried and poured onto a 20g SCX3 cartridge and eluted with methanol (4x20ml) and 1M ammonia solution (4x20ml). The combined ammonia washings were evaporated to dryness and the residue was chromatographed on a Bond Elut column eluting with 20% methanol in ethyl acetate to give the title compound, yield 44mg. MH⁺ 602. NMR (DMSO d6): 1-1.8 (m, 11H) 1.9-2.4 (m, 7H) 2.8 (s, 3H) 3.2-3.6 (m, 5H) 4.4 (d, 2H) 5.9 (d, 1H) 6.6 (m, 1H) 7.1-7.2 (m, 5H) 7.5 (d, 2H) 7.85 (d, 2H) 8.6 (t, 1H).

### (3R) 3-[1-Hydroxy-3-phenyl-3-(N-methanesulphonylpiperidin-4-yl)propyl]-(4R,5S)-1,5-dimethyl-4-phenyl-2-imidazolidinone.

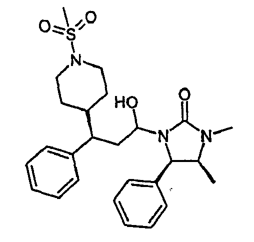

Diisobutylaluminium hydride (8.2ml) was added dropwise over 15 minutes to a solution of 3R 3-[3-phenyl-3-(N-methanesulphonylpiperidin-4-yl)propionyl]-(4R,5S)-1,5-dimethyl-4-phenyl-2-imidazolidinone (2g) in dichloromethane (75ml) at -78°C and the mixture was stirred at this temperature for 30 minutes. Saturated sodium sulphate solution (10ml) was added and the reaction mixture was allowed to warm to room temperature. The reaction mixture was filtered and the filtrate was washed with water (2x50ml) and dried. An aliquot of the dichloromethane solution was used for the next stage.

### (3R) 3-[3-Phenyl-3-(N-methanesulphonylpiperidin-4-yl)propionyl]-(4R,SS)-1,5-dimethyl-4-phenyl-2-imidazolidinone

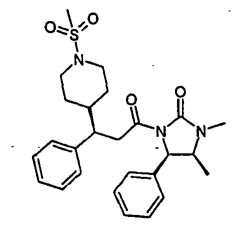

Methanesulphonyl chloride (1.2g) was added to a solution of (3R) 3-[3-phenyl-3-(piperidin-4-yl)propionyl]-(4R,5S)-1,5-dimethyl-4-phenyl-2-imidazolidinone (4.2g) and triethylamine (1.05g) in dichloromethane (100 ml) and the reaction mixture was stirred for 16 hours then washed with saturated aqueous sodium bicarbonate solution and dried. The residue obtained on removal of the solvent was triturated with diethyl ether (50ml) and the solid obtained was filtered and dried under vacuum at 40°C. Yield 3.2g. NMR (DMSO d6): 0.6 (d, 3H) 1-1.9 (m, 5H) 2.7 (s, 3H) 2.8 (s, 3H) 2.9-3.2 (m, 2H) 3.4-3.7 (m,3H) 3.7-3.9 (m, 2H) 5.2 (d, 1H) 6.8 (m, 2H) 7-7.4 (m, 8H).

### (3R) 3-[3-Phenyl-3-(piperidin-4-yl)propionyl]-(4R,5S)-1,5-dimethyl-4-phenyl-2-imidazolidinone.

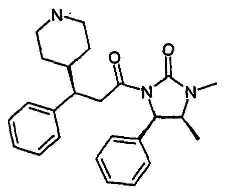

A mixture of (3R) 3-[3-phenyl-3-(N-benzyloxycarbonylpiperidin-4-yl)proplonyl]-(4R,5S)-1,5-dimethyl-4-phenyl-2-imidazolidinone (5g) and 20% Pd/C catalyst in ethanol (250ml) was hydrogenated using a hydrogen filled balloon. The catalyst was filtered and the filtrate evaporated to dryness to give the title compound, yield 4.2g.

### (R) 3-[3-Phenyl-3-(benzyloxycarbonylpiperidin-4-yl)propionyl]-(4R,5S)-1,5-dimethyl-4-phenyl-2-imidazolidinone.

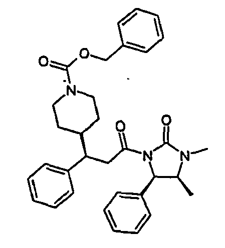

*N*,*N*,*N*',*N*'-Tetramethylethylenediamine (2.4g) was added to a suspension of cuprous iodide (4.02g) in THF (100 ml) and the mixture was stirred at room temperatutre for 30 minutes. The reaction mixture was cooled to -78°C and phenylmagnesium bromide (11.69ml of a 1M solution in THF) was added and the mixture was stirred at -78°C for 30 minutes. Dibutylboron triflate (11.69ml, 1M solution in diethyl ether) was added to a solution of 3-[3-(benzyloxycarbonylpiperidin-4-yl)acryloyl]-(4R,5S)-1,5-dimethyl-4-phenyl-2-imidazolidinone (4.9g) in THF (50ml) and this mixture was added dropwise over 10 minutes to the solution of the cuprate reagent. The reaction mixture was stirred at -78°C for 1 hour then allowed to warm to ambient temperature. The solvent was evaporated, the residue was dissolved in ethyl acetate and filtered through silica (100g). The ethyl acetate solution was washed with 2M HCl (1x100ml), dried and evaporated to dryness. The residue was passed down a Bond-Elut column eluted with a mixture of ethyl acetate and isohexane (1:1) to give the title compound as a single diastereoisomer by NMR. Yield 5.1g. NMR (DMSOd6): 0.5 (d, 3H) 0.8-1.1 (m.2H) 1.3 (d, 1H) 1.7 (m, 2H) 2.6 (m, 5H) 2.85-3.1 (m, 4H) 5.05 (s, 2H) 5.2 (d, 1H) 6.8 (m, 2H) 7.1-7.5 (m, 13H).

### 3-[3-(Benzyloxycarbonylpiperidin-4-yl)acryloyl]-(4R,5S)-1,5-dimethyl-4-phenyl-2-imidazolidinone

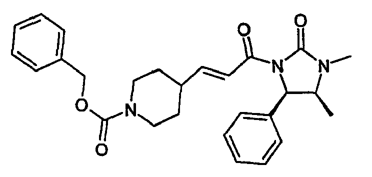

1-Chloro-N,N,2-trimethyl-1-propenylamine (1.37g) was added dropwise over 10 minutes to a solution of 3-(benzyloxycarbonylpiperidin-4-yl)propenoic acid (2.5g) in THF (20ml) and the mixture was stirred for 1.5 hours. Lithium bis(trimethylsilyl)amide (8.65ml)was added to a solution of (4R,5S)-1,5-dimethyl-4-phenyl-2-imidazolidinone (1.64g) in THF (20ml) at -10°C and the mixture was stirred at -10°C for 10 minutes, allowed to warm to 0°C and then cooled again to -10°C. The acid chloride solution (prepared above) was added dropwise and the mixture was allowed to warm to room temperature. The reaction mixture was poured into water (100ml) and extracted with ethyl acetate (3x50ml). The combined extracts were dried, evaporated to dryness and the residue was chromatographed on a Bond-Elut column eluted with an ethyl acetate/isohexane mixture (1:1) to give the title compound, yield 3.6g. NMR (DMSOd6): 0.6 (d, 3H) 0.95 (d, 1H) 1.2 (m, 2H) 1.55 (m, 2H) 2.4 (m, 1H) 2.3 (s, 3H) 2.8 (m, 2H) 3.95 (m, 3H) 5 (s, 2H) 5.3 (d, 1H) 6.9 (m, 1H) 7.1 (m, 2H) 7.2-7.4 (m, 8H).

### 3-(Benzyloxycarbonylpiperidin-4-yl)propenoic acid

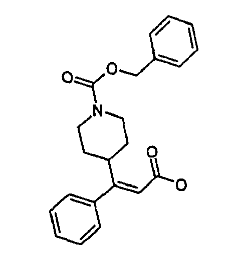

A mixture of N-benzyloxycarbonyl-4-formylpiperidine (10g), malonic acid (4.2g), pyridine (4ml) and piperidine (0.4ml) was heated at 100°C for 2 hours. The reaction mixture was allowed to cool and was diluted with ethyl acetate (100ml). The solution was washed with 2M HCl (2x100ml), dried and evaporated to dryness. The residue was triturated with isohexane to give the title compound, yield 13.5g. NMR (DMSOd6): 1.2 (m, 2H) 1.7 (m, 2H) 2.35 (m, 1H) 2.85 (m, 2H) 4 (d, 2H) 5.05 (s, 2H) 5.75 (d, 1H) 6.75 (m, 1H) 7.35 (m, 5H) 12.25 (broad peak, 1H).

### EXAMPLE 5

This Example illustrates the preparation of (S) N-(4-methanesulphonylphenyl)-1-[3-phenyl-3-(4-methanesulphonylphenyl)-propyl]piperidin-4-yl acetamide (Compound No. 1 Table IV).

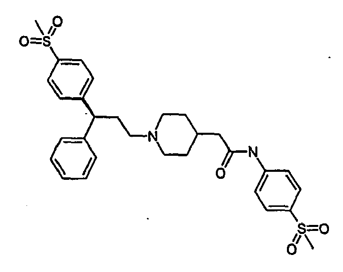

Sodium triacetoxyborohydride (112mg) was added to a mixture of N-(4-methanesulphonylphenyl)piperidin-4-yl acetamide (103mg) and (S) 3-phenyl-3-(4-methanesulphonylphenyl)propionaldehyde (100mg) in dichloromethane (20ml) containing 2 drops of acetic acid and the reaction mixture was stirred for 16 hours. The reaction mixture was washed with 2M sodium hydroxide (15ml) and brine (15ml) and dried. The solvent was removed and the residue was chromatographed on a 20g Bond Elut column eluting with a solvent gradient of ethyl acetate- 20% methanol/ethyl acetate to give the title compound ,yield 150mg, MH⁺ 569.

### EXAMPLE 6

This Example illustrates the preparation of *N*-(4-fluorophenyl)-2-[1-((3*S*)-3-phenyl-3-{4-[(trifluoromethyl)sulfonyl]piperazin-1-yl}propyl)piperidin-4-yl]acetamide (Compound 9 Table III)

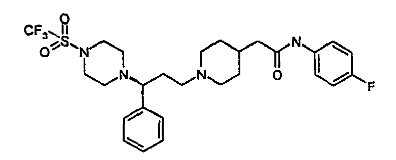

Triethylamine (0.16 ml) and trifluoromethanesulphonylpiperazine hydrochloride (114 mg) were added to a solution of 2-{1-[(3*R*)-3-chloro-3-phenylpropyl]piperidin-4-yl}-*N*-[4-fluorophenyl]acetamide (221 mg) in dichloromethane (15 ml) and the mixture was allowed to stand for 72 hours. The reaction mixture was washed with water (20 ml) and brine (20 ml) and was poured onto a 20g silica Bond-elut column and was eluted with a solvent gradient of ethyl acetate- 20% methanol/ethyl acetate to give the title compound, yield 152 mg, M+H 571.

¹H NMR (CDCl₃): 1.3 (m, 2H) 1.8 (m, 3H) 1.9 (m, 3H) 2.1 (m, 2H) 2.3 (m, 2H) 2.5 (m, 3H) 2.8-3.0 (m,3H) 3.4-3.6 (m, 6H) 7.0 (m, 2H) 7.2 (m, 2H) 7.3 (m, 4H) 7.6 (m, 2H).

### Method A

### (S)-3-Phenyl-3-(4-methanesulfonylphenyl)propionaldehyde

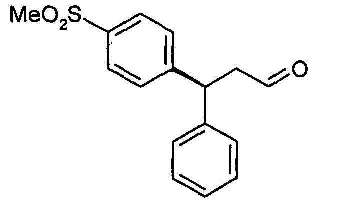

### Step 1: Preparation of (4R, 5S)-1-[(S)-3-(4-methanesulfonyl-phenyl)-3-phenyl-propionyl]-3,4-dimethyl-5-phenyl-imidazolidin-2-one

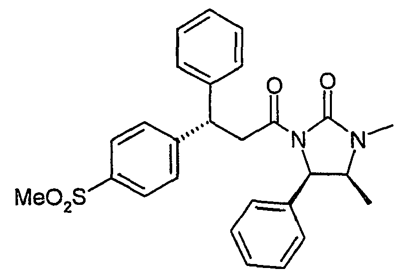

To a mixture of copper (I) iodide (960mg, 5.0mmol) and THF (20mL) was added *N*,*N*,*N*',*N*'-tetramethylethylenediamine (0.83mL, 5.5mmol) and the resulting mixture was stirred at room temperature for 10min. then cooled to -78°C. Phenylmagnesium bromide (5.0mL, 1M in THF, 5.0mmol) was added and the resulting mixture stirred at -78°C for 15min. A solution of di-n-butylboron triflate (3.0mL, 1M in diethyl ether, 3.0mmol) and (*E*)-(4*R*, 5*S*)-1-(3-[4-methanesulfonylphenyl]acryloyl)-3,4-dimethyl-5-phenyl-imidazolidin-2-one (1.0g, 2.51mmol)(prepared according to step 4 ) in THF (15mL) was added and the resulting mixture was stirred whilst allowing to warm to room temperature for 18h. The reaction mixture was washed with saturated aqueous ammonium chloride, water and brine, dried (MgSO₄) and evaporated. The residue was purified by eluting through a 20g Bond Elut with gradient of isohexane to ethyl acetate giving the sub-titled compound (1.49g, 100%); NMR (CDCl₃): 0.78 (d, 3H), 2.82 (s, 3H), 3.00 (s, 3H), 3.78 (dd, 1H), 3.80 (m, 1H), 3.98 (dd, 1H), 4.72 (m, 1H), 5.19 (d, 1H), 6.99 (m, 2H), 7.22 (m, 8H), 7.48 (d, 2H), 7.79 (d, 2H); MS: 477 (MH+).

### Step 2: Preparation of (S)-3-phenyl-3-(4-methanesulfonylphenyl)propan-1-ol

To a solution of (4*R*, 5*S*)-1-[(*S*)-3-(4-methanesulfonyl-phenyl)-3-phenyl-propionyl]-3,4-dimethyl-5-phenyl-imidazolidin-2-one (846mg, 1.78mmol) in THF (20mL) at 0°C was added lithium aluminium hydride (3.6mL, 1M in THF, 3.6mmol) and the resulting mixture was stirred for 15min. The reaction was quenched by the addition of 2M aqueous sodium hydroxide. The phases were separated and the organic phase pre-absorbed onto a Bond Elut and eluted with a gradient of isohexane to ethyl acetate giving the sub-titled compound as a white solid (285mg, 55%); NMR (CDCl₃): 1.63 (br s, 1H), 2.33 (m, 2H), 3.00 (s, 3H), 3.59 (t, 2H), 4.28 (t, 1H), 7.23 (m, 5H), 7.43 (d, 2H), 7.82 (d, 2H).

### Step 3: Preparation of the title compound

To a solution of (*S*)-3-phenyl-3-(4-methanesulfonylphenyl)propan-1-ol (244mg, 0.84mmol) in DCM (5mL) was added Dess-Martin periodinane (392mg, 0.92mmol) and the resulting mixture was stirred at room temperature for 1.5h. The mixture was washed with 2M aqueous sodium hydroxide (2 x 10mL), dried and evaporated to give the title compound.

### Step 4: Preparation of E-(4R, 5S)-1-(3-[4-Methanesulphonylphenyl]acryloyl)-3,4-dimethyl-5-phenyl-imidazolidin-2-one

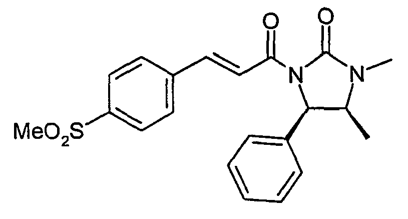

To a stirred solution of 3-(4-methanesulphonylphenyl)acrylic acid (7.14g, 31.5mmol) in DCM (10mL) was added thionyl chloride (3mL, 34.7mmol) dropwise and the resulting mixture was stirred at room temperature for 18h. To this solution was added DIPEA (5.04mL, 28.9mmol) dropwise at room temperature. The resulting solution was added to a stirred solution of (4*R*, 5*S*)-3,4-dimethyl-5-phenyl-imidazolidin-2-one (5.0g, 26.3mmol) in DCM (20mL) and DIPEA (4.58mL, 26.9mmol) and the resulting mixture stirred at room temperature for 4h. The mixture was washed with water and brine, pre-absorbed onto a Bond Elut and eluted with a gradient of isohexane to ethyl acetate giving the title compound as a solid (7.61g, 73%); NMR (CDCl₃): 0.84 (d, 3H), 2.89 (s, 3H), 3.04 (s, 3H), 3.98 (m, 1H), 5.42 (d, 1H), 7.20 (m, 2H), 7.32 (m, 3H), 7.69 (d, 1H), 7.74 (d, 2H), 7.93 (d, 2H), 8.31 (d, 1H); MS: 399 (MH+).

### Method B

### N-(4-Methanesulphonylphenyl)-piperidin-4-ylacetamide

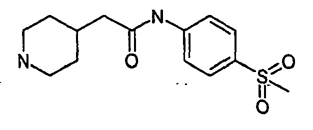

### Step 1 Preparation of N-(4-methylthiophenyl)-1-tert-butoxycarbonylpiperidin-4-ylacetamide

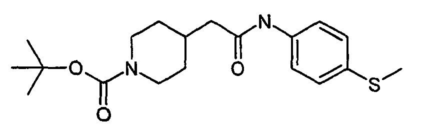

HATU (6.25g) was added to a solution of N-tert-butoxycarbonyl-4-piperidine acetic acid (4g) and N,N-diisopropylethylamine (5.72ml) in dichloromethane (50ml) and the mixture was stirred for 15 minutes. 4-Methylthioaniline (2.05ml) was added and the mixture was stirred for 1 hour and washed successively with 20 ml each of 1M HCl, saturated sodium bicarbonate, water, brine and dried. The residue obtained on removal of the solvent was chromatographed on a 70g silica Bond Elut column eluting with a solvent gradient of isohexane-30% ethyl acetate/isohexane. Yield 4.5g, MH⁺ 265.

### Step 2 Preparation of N-(4-methylsulphonylphenyl)-tert-butoxycarbonylpiperidin-4-ylacetamide

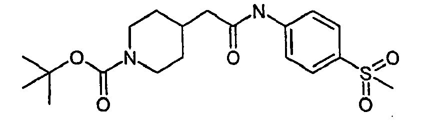

m-Chloroperbenzoic acid (6.4g) was added to a stirred solution of N-(4-methylthiophenyl)-1-tert-butoxycarbonylpiperidin-4-ylacetamide (4.7g) in dichloromethane (50ml) at 0 °C. The reaction mixture was allowed to warm to room temperature and was stirred for 2 hours, then washed with 2M sodium hydroxide (15ml) and brine (15ml) and dried. The oil obtained on removal of the solvent was used for the next step.

### Step 3 Preparation of the title compound

The oil from step 2 was dissolved in dichloromethane (50ml) and treated with trifluoroacetic anhydride (10ml). The reaction mixture was allowed to stand at room temperature for 1 hour and was evaporated to dryness. The residue obtained was dissolved in 2M sodium hydroxide (15ml) and extracted with dichloromethane (3x40ml), dried and evaporated to dryness to give the title compound, yield 1.92g, MH⁺297.

The following analogues were prepared using steps 1 and 3 described above and the appropriate anilines:

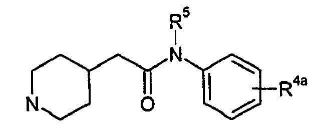

| Compound No | R⁵ | R^{4a} |
|---|---|---|
| 1 | H | 4-F |
| 2 | H | 3,5-diF |
| 3 | H | 4-Cl |
| 5 | H | 4-methyl |

### Method C

### 3-Phenyl-3-(N-methanesulphonylpiperidin-4-yl)propionaldehyde

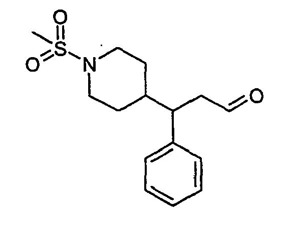

### Step 1 Preparation of 4-benzoyl-1-methanesulphonylpiperidine

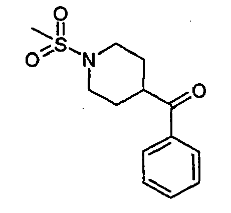

Methanesulphonyl chloride was added to a stirred slurry of 4-benzoylpiperidine hydrochloride (4.51g) and triethylamine (8.35ml) in dichloromethane (100ml) at 0°C. The reaction mixture was allowed to warm to room temperature and was stirred for 16 hours. The mixture was diluted with dichloromethane (50ml) and washed with ammonium chloride solution (2x25ml) and brine (25ml), dried and evaporated to dryness to give 4-benzoyl-1-methanesulphonylpiperidine as a white solid, yield 3.98g. NMR (CDCl₃): 1.93 (m, 4H) 2.81 (s, 3H) 2.98 (d-t, 2H) 3.40 (m, 1H) 3.77 (m, 2H) 7.43 (t, 2H) 7.57 (t, 1H) 7.89 (d, 2H).

### Step 2 Preparation of ethyl 3-phenyl-3-(N-methanesulphonylpiperidin-4-yl)acrylate.

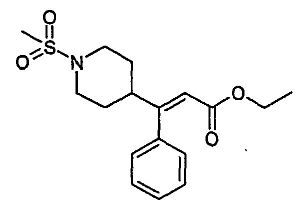

Lithium bis(trimethylsilyl)amide (16.3ml of a 1M solution in THF) was added dropwise to a solution of triethylphosphonoacetate (2.93ml) in THF at 0°C under an argon atmosphere and the mixture was stirred for 30 minutes. A slurry of 4-benzoyl-1-methanesulphonylpiperidine (3.96g) in THF (30ml) was added, the reaction mixture was allowed to warm to room temperature and stirring was continued for 24 hours. The reaction mixture was diluted with dichloromethane (80ml) and water (80ml). The organic layer was washed with water and the combined aqueous extracts were in turn extracted with dichloromethane (50ml). The combined dichloromethane extracts were washed with brine (25ml), dried and evaporated to dryness. The residue was chromatographed on a 90g Biotage column (available from Biotage UK Ltd, Foxhole business Park, Hertford) eluted with a solvent gradient (30-5% ethyl acetate/isohexane to give a less polar fraction (1.62g) and a more polar fraction (0.53g). Both fractions (cis/trans isomers) were combined and used for the next step.
Less polar NMR (CDCl₃): 1.27 (t, 3H) 1.69 (m, 2H) 1.81 (d, 2h) 2.72 (s, 3H) 2.72 (t, 2H) 3.81 (d, 2H) 3.88 (m, 1H) 4.21 (q,2H) 5.78 (s, 1H) 7.11 (m, 2H) 7.27 (m, 3H)
More polar NMR (CDCl₃): 1.01 (t, 3H) 1.56 (m, 2H) 1.85 (d, 2H) 2.31 (m, 1H) 2.63 (t, 2H) 2.74 (s, 3H) 3.83 (d, 2H) 3.92 (q, 3H) 5.82 (s, 1H) 7.04 (d, 2H) 7.30 (m, 3H).

### Step 3 Preparation of ethyl 3-phenyl-3-(N-methanesulphonylpiperidin-4-yl)propionate

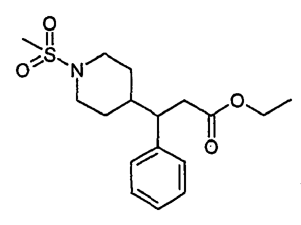

A solution of ethyl 3-phenyl-3-(N-methanesulphonylpiperidin-4-yl)acrylate (2.06g) in ethanol (30ml) was hydrogenated over 24 hours under a hydrogen filled balloon using 20% palladium hydroxide as catalyst. The reaction mixture was filtered through Celite and the filtrate evaporated to dryness. The product obtained was used for the next step without further purification. MH⁺340.

### Step 4 3-Phenyl-3-(N-methanesulphonylpiperidin-4-yl)propan-1-ol.

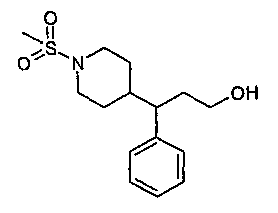

A solution of ethyl 3-phenyl-3-(N-methanesulphonylpiperidin-4-yl)propionate (2g) in THF (10ml) was added to a suspension of lithium aluminium hydride (232mg) in THF (20ml) at 0°C under argon over 30 minutes. The reaction mixture was allowed to warm to room temperature and stirred for 2 hours. Water (10ml) was added followed by magnesium sulphate (10g). The reaction mixture was filtered and the filtrate evaporated to dryness to give the product as a white foam, yield 1.57g. NMR (CDCl₃): 1.40 (m, 4H) 1.57 (m,1H) 1.78 (m, 1H) 2.01 (m, 2H) 2.45 (m, 2H) 2.58 (t, 1H) 2.70 (m, 3H) 3.31 (m, 1H) 3.42 (m, 1H) 3.67 (d, 1H) 3.80 (d, 1H) 7.04 (d, 1H) 7.19 (t, 1H) 7.29 (q, 2H).

### Step 5 Preparation of the title compound

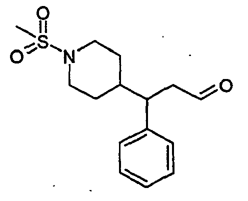

Dess-Martin periodinane (739mg) was added to a stirred solution of 3-phenyl-3-(N-methanesulphonylpiperidin-4-yl)propan-1-ol (454mg) in dichloromethane (8ml) and stirring was continued for 2 hours. The reaction mixture was diluted with dichloromethane (100ml) and washed with 2M sodium hydroxide (2x50ml), brine (50ml) and dried. The product obtained on removal of the solvent was used in subsequent steps without purification.

### Method D

### Preparation of 2-{1-[(3R)-3-chloro-3-phenylpropyl]piperidin-4-yl}-N-[4-fluorophenyl]acetamide

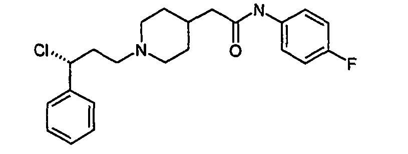

### Step 1 Preparation of 2-{1-[(3S)-3-hydroxy-3-phenylpropyl]piperidin-4-yl}-N-[4-fluorophenyl]acetamide.

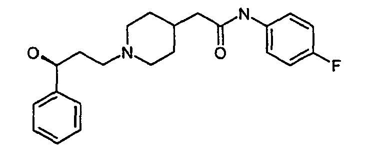

A solution of (3*S*)-3-hydroxy-3-phenylpropyl 4-methylbenzenesulfonate (844 mg, CAS No. 156453-52-0) in 1,4-dioxan (5 ml) was added to a mixture of *N*-(4-fluorophenyl)-2-piperidin-4-ylacetamide hydrochloride (750 mg, prepared according to Method B, M+H 237), triethylamine (0.38 ml) and potassium carbonate (572 mg) in 1,4-dioxan (15 ml) and the mixture was stirred and heated at 90°C for 4 hours. The reaction mixture was evaporated to dryness and the residue was dissolved in dichloromethane (20 ml) and washed with water (20 ml) and brine (20 ml) and dried. The solvent was removed and the residue was purified by chromatography on a 20g silica Bond-elut column using a solvent gradient of ethyl acetate-20% methanol/ethyl acetate. Yield 733 mg, M+H 371.

### Step 2

Methanesulphonyl chloride (0.159 ml) was added to a solution of 2-{1-[(3*S*)-3-hydroxy-3-phenylpropyl]piperidin-4-yl}-*N*-[4-fluorophenyl]acetamide (723 mg) in dichloromethane (10 ml) containing triethylamine (0.326 ml) and the mixture was stirred for 1 hour then washed with water (20 ml) and brine (20 ml) and dried. Removal of the solvent gave the title compound as an off-white foam, yield 442 mg, M+H 389.

### EXAMPLE 7

The ability of compounds to inhibit the binding of RANTES was assessed by an *in vitro* radioligand binding assay. Membranes were prepared from Chinese hamster ovary cells which expressed the recombinant human CCR5 receptor. These membranes were incubated with 0.1nM iodinated RANTES, scintillation proximity beads and various concentrations of the compounds of the invention in 96-well plates. The amount of iodinated RANTES bound to the receptor was determined by scintillation counting. Competition curves were obtained for compounds and the concentration of compound which displaced 50% of bound iodinated RANTES was calculated (IC₅₀). Preferred compounds of formula (I) have an IC₅₀ of less than 50µM.

### EXAMPLE 8

The ability of compounds to inhibit the binding of MIP-1α was assessed by an *in vitro* radioligand binding assay. Membranes were prepared from Chinese hamster ovary cells which expressed the recombinant human CCR5 receptor. These membranes were incubated with 0.1nM iodinated MIP-1α, scintillation proximity beads and various concentrations of the compounds of the invention in 96-well plates. The amount of iodinated MIP-1α bound to the receptor was determined by scintillation counting. Competition curves were obtained for compounds and the concentration of compound which displaced 50% of bound iodinated MIP-1α was calculated (IC₅₀). Preferred compounds of formula (I) have an IC₅₀ of less than 50µM.

Results from this test for certain compounds of the invention are presented in Table IV. In Table IV the results are presented as Pic50 values. A Pic50 value is the negative log (to base 10) of the IC₅₀ result, so an IC50 of 1µM (that is 1 x 10⁻⁶M) gives a Pic50 of 6. If a compound was tested more than once then the data below is an average of the probative tests results.

| Table number | Compound number | Pic50 |
|---|---|---|
| I | 3 | 7.92 |
| I | 5 | 8.88 |
| I | 6 | 8.25 |
| IV | 1 | 9.06 |
| V | 3 | 7.05 |

### Scheme 1

To prepare compounds of the invention, for example wherein R¹ is aryl or C-linked piperidine. 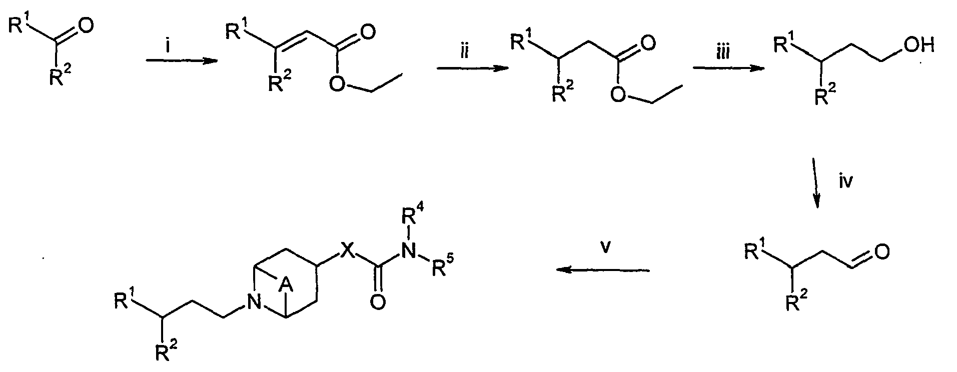
- i: Wittig reaction (eg LHDMS, triethylphosphonoacetate)
- ii: Catalytic hydrogenation (eg H₂, 10% Pd/C)
- iii: Reduction (eg lithium aluminium hydride)
- iv: Oxidation (eg Dess-Martin oxidation)
- v: reductive amination with 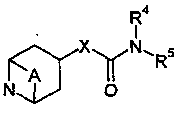 (eg using sodium triacetoxyborohydride)

### Scheme 2

To prepare compounds of the invention, for example wherein R¹ is aryl or C-linked piperidine. 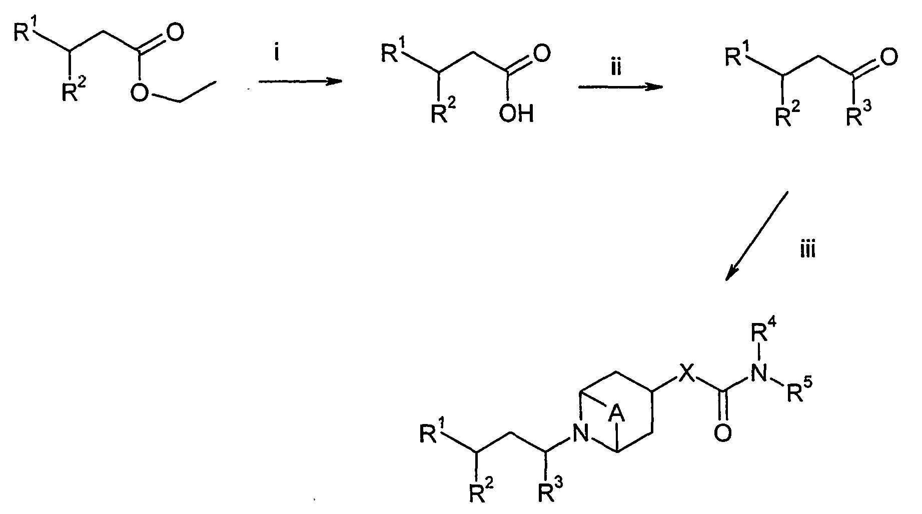
- i: Base hydrolysis (eg LiOH, MeOH/H₂O)
- ii: MeMgCl, R³MgBr, Et₂O
- iii: reductive amination 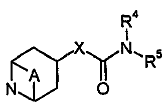 in presence of titanium tetraisopropoxide (eg using sodium triacetoxyborohydride)

### Scheme 3

To prepare compounds of the invention, for example wherein R¹ is aryl or heterocyclyl. 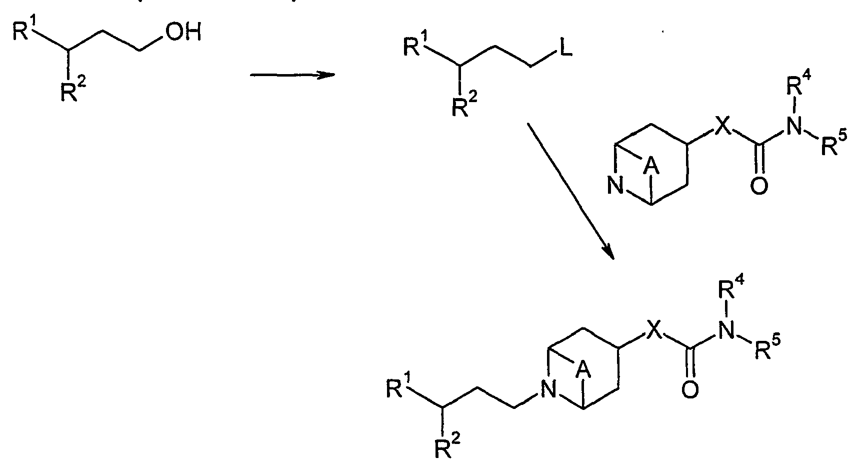 wherein L is an activated group such as halogen, mesylate, tosylate or triflate.

### Scheme 5

To prepare compounds of the invention, for example wherein R¹ is piperazine 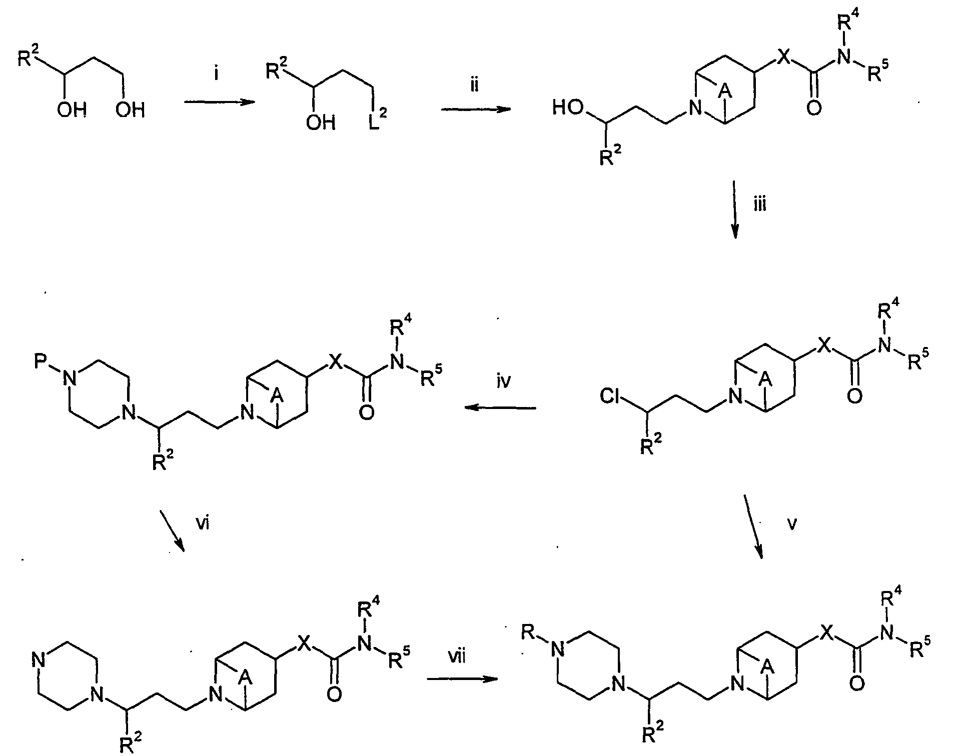
- i: Conversion of an OH to a leaving group (eg tosyl chloride (L² is Tosylate) or mesyl chloride (L² is Mesylate))
- ii: displacement reaction with 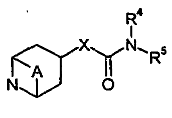 (eg in presence of triethylamine)
- iii: Mesyl chloride, DCM 0°C
- iv: Displacement reaction with mono-protected piperazine (P = protecting group)
- v: Displacement reaction with R substituted piperazine
- vi: Deprotection (TFA for Boc, hydrogenation for Cbz)
- vii: Depending on R, acylation, sulphonylation, alkylation, reductive amination

### Scheme 6

To prepare compounds of the invention, for example wherein R¹ is aryl or piperidine. 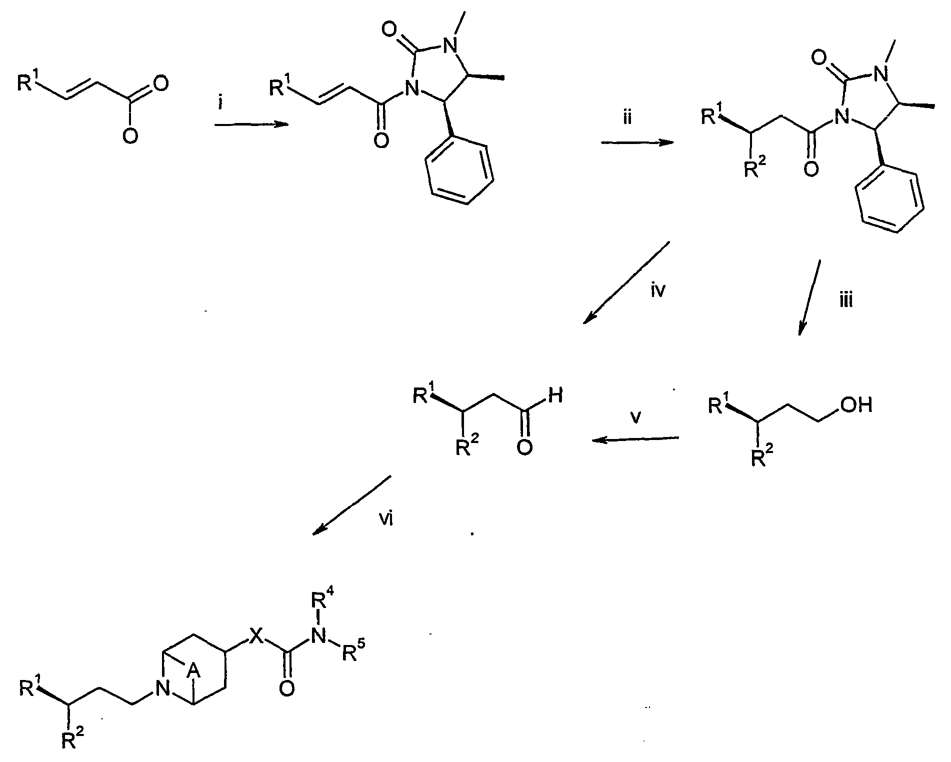
- i: activation of acid group and coupling with chiral auxiliary (eg SOCl₂, 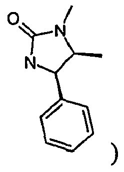
- ii: 1,4-addition of organocuprate (eg R²MgBr, Cu(I)I, TMEDA, di-butylboron triflate)
- iii: reduction (eg LAH)
- iv: Dibal
- v: Oxidation (eg Dess-Martin reagent)
- vi: reductive amination (eg with sodium triacetoxyborohydride)

### Scheme 7

To prepare compounds of the invention. 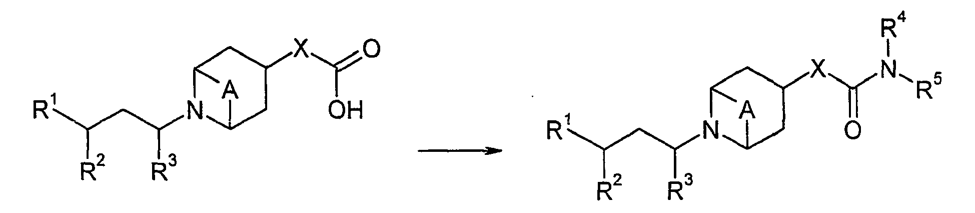 activation of acid group and coupling with R⁴R⁵NH.

## Claims

1. A compound of formula (I): 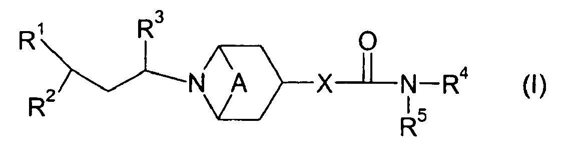 wherein
A is absent or is (CH₂)₂;
R¹ is heterocyclyl wherein the heterocyclyl group is selected from pyran, piperidine, piperazine, pyrrolidine or azetidine and R¹ is optionally mono-substituted by C₁₋₆ alkyl, C₃₋₇ cycloalkyl, phenyl {optionally substituted by halo, C₁₋₄ alkyl, C₁₋₄ alkoxy, CF₃ or OCF₃}, S(O)₂(C₁₋₄ alkyl), S(O)₂(C₁₋₄ fluoroalkyl), S(O)₂phenyl {optionally substituted by halo, cyano, C₁₋₄ alkyl, C₁₋₄ alkoxy, CF₃, OCF₃, S(O)₂(C₁₋₄ alkyl) or S(O)₂(C₁₋₄ fluoroalkyl)}, benzyl {optionally substituted by halo, C₁₋₄ alkyl, C₁₋₄ alkoxy, CF₃ or OCF₃}, C(O)H, C(O) (C₁₋₄ alkyl), benzoyl {optionally substituted by halo, C₁₋₄ alkyl, C₁₋₄ alkoxy, CF₃ or OCF₃}, C(O)₂(C₁₋₄ alkyl), C(O)NH₂, C(O)NH(C₁₋₄ alkyl) or C(O)NHphenyl {optionally substituted by halo, C₁₋₄ alkyl, C₁₋₄ alkoxy, CF₃ or OCF₃}, or R¹ is phenyl optionally substituted by S(O)₂C₁₋₄ alkyl;
R² is phenyl optionally substituted by halo, C₁₋₄ alkyl, C₁₋₄ alkoxy, S(O)ₙ(C₁₋₄ alkyl), nitro, cyano or CF₃; wherein n is 0, 1 or 2;
R³ is H;
R⁴ is phenyl or benzyl, either of which is optionally substituted by halo, C₁₋₄ alkyl, C₁₋₄ alkoxy, S(O)_{z}(C₁₋₄ alkyl), nitro, cyano or CF₃; wherein z is 0, 1 or 2;
R⁵ is H;
X is CH₂, (CH₂)₂, CH=CH, OCH₂ or S(O)ₙCH₂;
n is 0, 1 or 2;
or a pharmaceutically acceptable salt thereof or a solvate thereof.

2. A compound as claimed in claim 1 wherein A is absent.

3. A compound as claimed in claim 1 or 2 wherein R¹ is piperidinyl or piperazinyl substituted by S(O)₂C₁₋₄ alkyl, S(O)₂C₁₋₄fluoroalkyl or C(O)NH-phenyl.

4. A compound as claimed in claim 1 or 2 wherein R¹ is phenyl substituted by S(O)₂C₁₋₄ alkyl.

5. A compound as claimed in claim 1, 2 or 3 wherein R² is phenyl optionally substituted by 0, 1 or 2 fluorines.

6. A compound as claimed in any one of the preceding claims wherein X is CH₂ or CH=CH.

7. A process for the preparation of a compound of formula (I) as claimed in claim 1 wherein:
a. for a compound of the invention wherein R¹ is an N-linked optionally substituted heterocycle, reacting a compound of formula (II): 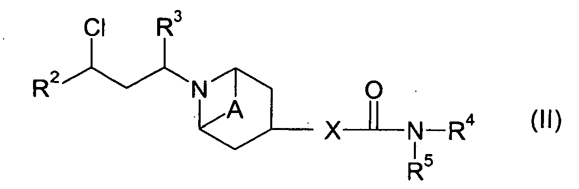 wherein R², R³, R⁴, R⁵, A and X are as defined in claim 1, with a compound R¹H (wherein the H is on a heterocycle ring nitrogen atom) wherein R¹ is as defined in claim 1, in the presence of a suitable base, in a suitable solvent;
b. for a compound of the invention wherein R³ is hydrogen, coupling a compound of formula (III): 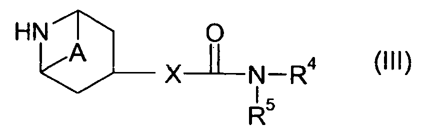 wherein R⁴, R⁵, A and X are as defined in claim 1, with a compound of formula (IV): 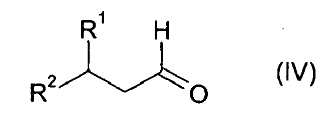 wherein R¹ and R² are as defined in claim 1, in the presence of NaBH(OAc)₃ (wherein Ac is C(O)CH₃) in a suitable solvent at room temperature;
c. activating the acid group of a compound of formula (V) 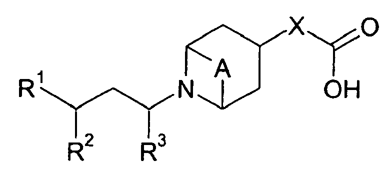 wherein X, A, R¹, R² and R³ are as defined in claim 1, and coupling the product so formed with an amine R⁴R⁵NH (wherein R⁴ and R⁵ are as defined in claim 1).

8. A pharmaceutical composition which comprises a compound as claimed in claim 1, or a pharmaceutically acceptable salt thereof or solvate thereof, and a pharmaceutically acceptable adjuvant, diluent or carrier.

9. A compound as claimed in claim 1, or a pharmaceutically acceptable salt thereof or solvate thereof, for use as a medicament.

10. Use of a compound of the formula (I) as claimed in claim 1 for the manufacture of a medicament for the treatment of transplant rejection, respiratory disease, psoriasis or rheumatoid arthritis.

## Patentansprüche

1. Verbindung der Formel (I): 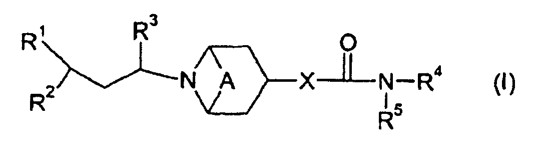 worin
A fehlt oder für (CH₂) steht;
R¹ für Heterocyclyl steht, wobei die Heterocyclylgruppe unter Pyran, Piperidin, Piperazin oder Azetidin ausgewählt ist und R¹ gegebenenfalls einfach durch C₁₋₆-Alkyl, C₃₋₇-Cycloalkyl, Phenyl {gegebenenfalls substituiert durch Halogen, C₁₋₄₋Alkyl, C₁₋₄-Alkoxy, CF₃ oder OCF₃}, S(O)₂(C₁₋₄₋Alkyl), S(O)₂(C₁₋₄-Fluoralkyl), S(O)₂-Phenyl {gegebenenfalls substituiert durch Halogen, Cyano, C₁₋₄-Alkyl, C₁₋₄-Alkoxy, CF₃, OCF₃, S(O)₂(C₁₋₄-Alkyl) oder S(O)₂(C₁₋₄-Fluoralkyl)}, Benzyl {gegebenenfalls substituiert durch Halogen, C₁₋₄-Alkyl, C₁₋₄-Alkoxy, CF₃ oder OCF₃}, C(O)H, C(O) (C₁₋₄-Alkyl), Benzoyl {gegebenenfalls substituiert durch Halogen, C₁₋₄₋Alkyl, C₁₋₄-Alkoxy, CF₃ oder OCF₃}, C(O)₂(C₁₋₄₋Alkyl), C(O)NH₂, C(O)NH(C₁₋₄-Alkyl) oder C(O)NH-Phenyl (gegebenenfalls substituiert durch Halogen, C₁₋₄-Alkyl, C₁₋₄-Alkoxy, CF₃ oder OCF₃} substituiert ist, oder R¹ für gegebenenfalls durch S(O)₂(C₁₋₄₋Alkyl) substituiertes Phenyl steht;
R² für gegebenenfalls durch Halogen, C₁₋₄-Alkyl, C₁₋₄-Alkoxy, S(O)ₙ(C₁₋₄-Alkyl), Nitro, Cyano oder CF₃ substituiertes Phenyl steht, wobei n 0, 1 oder 2 bedeutet;
R³ für H steht;
R⁴ für jeweils gegebenenfalls durch Halogen, C₁₋₄₋Alkyl, C₁₋₄-Alkoxy, S(O)_{z}(C₁₋₄-Alkyl), Nitro, Cyano oder CF₃ substituiertes Phenyl oder Benzyl steht, wobei z 0, 1 oder 2 bedeutet;
R⁵ für H steht;
X für CH₂, (CH₂)₂, CH=CH, OCH₂ oder S(O)ₙCH₂ steht;
n für 0, 1 oder 2 steht;
oder ein pharmazeutisch annehmbares Salz davon oder ein Solvat davon.

2. Verbindung nach Anspruch 1, in der A fehlt.

3. Verbindung nach Anspruch 1 oder 2, in der R¹ für durch S(O)₂(C₁₋₄-Alkyl), S(O)₂(C₁₋₄-Fluoralkyl) oder C(O)NH-Phenyl substituiertes Piperidinyl oder Piperazinyl steht.

4. Verbindung nach Anspruch 1 oder 2, in der R¹ für durch S(O)₂(C₁₋₄-Alkyl) substituiertes Phenyl steht.

5. Verbindung nach Anspruch 1, 2 oder 3, in der R² für gegebenenfalls durch 0, 1 oder 2 Fluoratome substituiertes Phenyl steht.

6. Verbindung nach einem der vorhergehenden Ansprüche, in der X für CH₂ oder CH=CH steht.

7. Verfahren zur Herstellung einer Verbindung der Formel (I) nach Anspruch 1, bei dem man:
a. für eine erfindungsgemäße Verbindung, in der R¹ für einen N-verknüpften gegebenenfalls substituierten Heterocyclus steht, eine Verbindung der Formel (II) : 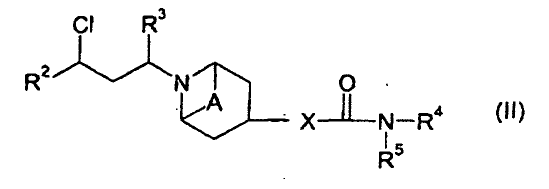 worin R², R³, R⁴, R⁵, A und X die in Anspruch 1 angegebene Bedeutung besitzen, in Gegenwart einer geeigneten Base in einem geeigneten Lösungsmittel mit einer Verbindung R¹H (worin das H sich an einem Ringstickstoffatom eines Heterocyclus befindet), worin R¹ die in Anspruch 1 angegebene Bedeutung besitzt, umsetzt;
b. für eine erfindungsgemäße Verbindung, in der R³ für Wasserstoff steht, eine Verbindung der Formel (III): 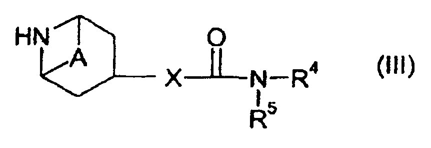 worin R⁴, R⁵, A und X die in Anspruch 1 angegebene Bedeutung besitzen, in Gegenwart von NaBH(OAc)₃ (worin Ac für C(O)CH₃ steht) in einem geeigneten Lösungsmittel bei Raumtemperatur mit einer Verbindung der Formel (IV): 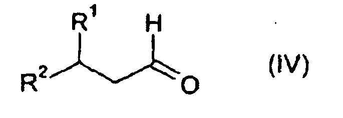 worin R¹ und R² die in Anspruch 1 angegebene Bedeutung besitzen, kuppelt;
c. die Säuregruppe einer Verbindung der Formel (V) 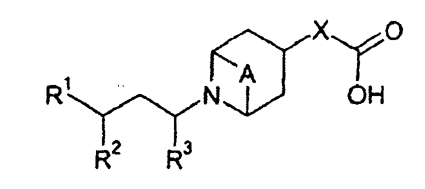 worin X, A, R¹, R² und R³ die in Anspruch 1 angegebene Bedeutung besitzen, aktiviert und das so gebildete Produkt mit einem Amin R⁴R⁵NH (worin R⁴ und R⁵ die in Anspruch 1 angegebene Bedeutung besitzen) kuppelt.

8. Pharmazeutische Zusammensetzung, enthaltend eine Verbindung nach Anspruch 1 oder ein pharmazeutisch annehmbares Salz davon oder Solvat davon und einen pharmazeutisch annehmbaren Hilfsstoff, ein pharmazeutisch annehmbares Verdünnungsmittel oder einen pharmazeutisch annehmbaren Träger.

9. Verbindung nach Anspruch 1 oder ein pharmazeutisch annehmbares Salz davon oder Solvat davon zur Verwendung als Arzneimittel.

10. Verwendung einer Verbindung der Formel (I) nach Anspruch 1 zur Herstellung eines Arzneimittels zur Behandlung von Transplantatabstoßung, Atemwegserkrankungen, Psoriasis oder rheumatoider Arthritis.

## Revendications

1. Composé de formule (I) : 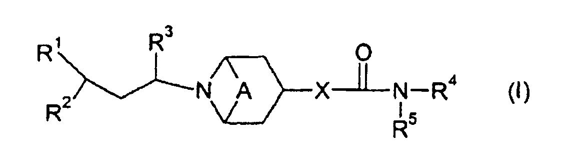 dans laquelle
A est absent ou est (CH₂)₂ ;
R¹ est hétérocyclyle où le groupement hétérocyclyle est choisi parmi pyrane, pipéridine, pipérazine, pyrrolidine et azétidine et R¹ est éventuellement mono-substitué par C₁₋₆ alkyle, C₃₋₇ cycloalkyle, phényle {éventuellement substitué par halogéno, C₁₋₄ alkyle, C₁₋₄ alcoxy, CF₃ ou OCF₃}, S(O)₂(C₁₋₄ alkyle), S(O)₂(C₁₋₄ fluoroalkyle), S(O)₂phényle {éventuellement substitué par halogéno, cyano, C₁₋₄ alkyle, C₁₋₄ alcoxy, CF₃, OCF₃, S(O)₂(C₁₋₄ alkyle) ou S(O)₂(C₁₋₄ fluoroalkyle)}, benzyle {éventuellement substitué par halogéno, C₁₋₄ alkyle, C₁₋₄ alcoxy, CF₃ ou OCF₃}, C(O)H, C(O)(C₁₋₄ alkyle), benzoyle {éventuellement substitué par halogéno, C₁₋₄ alkyle, C₁₋₄ alcoxy, CF₃ ou OCF₃}, C(O)₂(C₁₋₄ alkyle), C(O)NH₂, C(O)NH(C₁₋₄ alkyle) ou C(O) NHphényle {éventuellement substitué par halogéno, C₁₋₄ alkyle, C₁₋₄ alcoxy, CF₃ ou OCF₃}, ou R¹ est phényle éventuellement substitué par S(O)₂(C₁₋₄ alkyle) ;
R² est phényle éventuellement substitué par halogéno, C₁₋₄ alkyle, C₁₋₄ alcoxy, S(O)ₙ(C₁₋₄ alkyle), nitro, cyano ou CF₃, où n est 0, 1 ou 2 ;
R³ est H ;
R⁴ est phényle ou benzyle, dont l'un ou l'autre est éventuellement substitué par halogéno, C₁₋₄ alkyle, C₁₋₄ alcoxy, S(O)₂(C₁₋₄ alkyle), nitro, cyano ou CF₃, où z est 0, 1 ou 2 ;
R⁵ est H ;
X est CH₂, (CH₂)₂, CH=CH, OCH₂ ou S(O)ₙCH₂ ;
n est 0, 1 ou 2 ;
ou un sel pharmaceutiquement acceptable de celui-ci ou un solvate de celui-ci.

2. Composé selon la revendication 1, **caractérisé en ce que** A est absent.

3. Composé selon la revendication 1 ou 2, **caractérisé en ce que** R¹ est pipéridinyle ou pipérazinyle substitué par S(O)₂(C₁₋₄ alkyle), S(O)₂(C₁₋₄ fluoroalkyle) ou C(O)NH-phényle.

4. Composé selon la revendication 1 ou 2, **caractérisé en ce que** R¹ est phényle substitué par S(O)₂(C₁₋₄ alkyle).

5. Composé selon la revendication 1, 2 ou 3, **caractérisé en ce que** R² est phényle éventuellement substitué par 0, 1 ou 2 fluors.

6. Composé selon l'une quelconque des revendications précédentes, **caractérisé en ce que** X est CH₂ ou CH=CH.

7. Procédé de préparation d'un composé de formule (I) selon la revendication 1, **caractérisé en ce que** :
a. pour un composé de l'invention où R¹ est un hétérocycle éventuellement substitué lié par N, la réaction d'un composé de formule (II) : 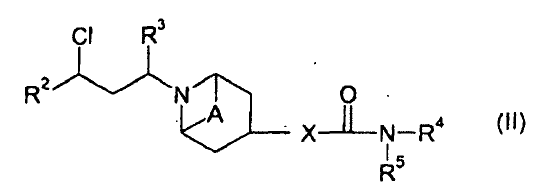 dans laquelle R², R³, R⁴, R⁵, A et X sont tels que définis dans la revendication 1, avec un composé R¹H (où le H est sur un atome d'azote du cycle hétérocyclique) où R¹ est tel que défini dans la revendication 1, en présence d'une base convenable, dans un solvant convenable ;
b. pour un composé de l'invention où R³ est hydrogène, le couplage d'un composé de formule (III) : 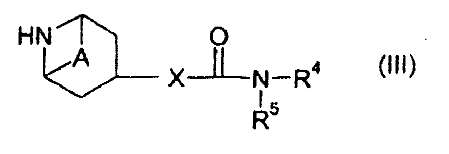 dans laquelle R⁴, R⁵, A et X sont tels que définis dans la revendication 1, avec un composé de formule (IV) : 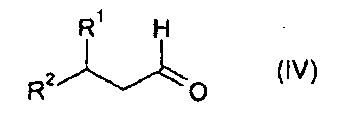 dans laquelle R¹ et R² sont tels que définis dans la revendication 1, en présence de NaBH(OAc)₃ (où Ac est C(O)CH₃) dans un solvant convenable, à température ambiante ;
c. l'activation du groupement acide d'un composé de formule (V) 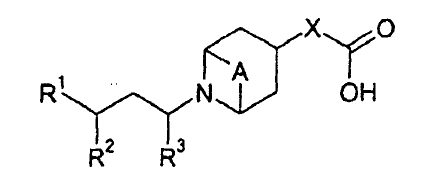 dans laquelle X, A, R¹, R² et R³ sont tels que définis dans la revendication 1, et le couplage du produit ainsi formé avec une amine R⁴R⁵NH (où R⁴ et R⁵ sont tels que définis dans la revendication 1).

8. Composition pharmaceutique, **caractérisée en ce qu'**elle comprend un composé selon la revendication 1, ou un sel pharmaceutiquement acceptable de celui-ci ou un solvate de celui-ci, et un adjuvant, un diluant ou un support pharmaceutiquement acceptable.

9. Composé selon la revendication 1, ou un sel pharmaceutiquement acceptable de celui-ci ou un solvate de celui-ci, destiné à être utilisé comme médicament.

10. Utilisation d'un composé de formule (I) selon la revendication 1, pour la fabrication d'un médicament destiné au traitement de rejet de greffe, de maladies respiratoires, de psoriasis ou de polyarthrite rhumatoïde.
